# EUROPEAN PATENT SPECIFICATION

(11) **EP 3 065 634 B1**
(45) Date of publication and mention of the grant of the patent: **27.03.2024**
(21) Application number: 14859965.7
(22) Date of filing: 06.11.2014
(51) Int. Cl.: A61B 5/24, A61N 1/00, A61B 90/00, A61B 17/00, A61B 17/02, A61B 5/00, A61B 5/296, A61B 5/389

(54) **NEUROPHYSIOLOGIC MONITORING DURING SPINE SURGERY**
NEUROPHYSIOLOGISCHE ÜBERWACHUNG WÄHREND EINER WIRBELSÄULENCHIRURGIE
SURVEILLANCE NEUROPHYSIOLOGIQUE PENDANT UNE CHIRURGIE DE LA COLONNE VERTÉBRALE

(30) Priority: 06.11.2013 US 201314073772
(43) Date of publication of application: 14.09.2016
(73) Proprietor: Nuvasive, Inc., San Diego, CA 92121 (US)
(72) Inventor: GHARIB, James, E., San Diego, CA 92121-5707 (US); FINLEY, Eric, San Diego, CA 92121-5707 (US); AZZARA, Adam, San Diego, CA 92121-5707 (US); NOVIKOV, Dmitry, San Diego, CA 92121-5707 (US); TAYLOR, William, San Diego, CA 92121-5707 (US)
(74) Representative: Morabito, Sara
(86) International application number: PCT/US2014/064449
(87) International publication number: WO 2015/069962

(56) References cited:
- WO-A1-2008/124079
- WO-A1-2010/044880
- WO-A1-2013/071309
- WO-A2-2006/084193
- US-A1- 2007 100 212
- US-A1- 2008 221 473
- DELETIS ET AL: "Intraoperative neurophysiological monitoring of the spinal cord during spinal cord and spine surgery: A review focus on the corticospinal tracts", CLINICAL NEUROPHYSIOLOGY, ELSEVIER SCIENCE, IE, vol. 119, no. 2, 22 December 2007 (2007-12-22), pages 248-264, XP022396854, ISSN: 1388-2457, DOI: 10.1016/J.CLINPH.2007.09.135
- SWASH, M ET AL.: 'Slowed motor conduction in lumbosacral nerve roots in cauda equina lesions: a new diagnostic technique.' JOURNAL OF NEUROLOGY, NEUROSURGERY, AND PSYCHIATRY. vol. 49, 1986, pages 808 3 - 816, XP055341315
- GONZALEZ, AA ET AL.: 'Intraoperative Neurophysiological Monitoring during Spine Surgery: A Review.' NEUROSURGICAL FOCUS. vol. 27, no. 4, 2009, page E6, XP055341316
- KAREN MINASSIAN ET AL: "Neuromodulation of lower limb motor control in restorative neurology", CLINICAL NEUROLOGY AND NEUROSURGERY, ELSEVIER, AMSTERDAM, NL, vol. 114, no. 5, 2 March 2012 (2012-03-02) , pages 489-497, XP028418326, ISSN: 0303-8467, DOI: 10.1016/J.CLINEURO.2012.03.013 [retrieved on 2012-03-10]

## Description

### FIELD

The present invention relates to a system generally aimed at surgery. More particularly, the present invention is directed at a system for performing neurophysiologic assessments during surgical procedures.

### BACKGROUND

The spinal column is a highly complex system of bones and connective tissues that provide support for the body and protect the delicate spinal cord and nerves. The spinal column includes a series of vertebral bodies stacked one atop the other, each vertebral body including an inner or central portion of relatively weak cancellous bone and an outer portion of relatively strong cortical bone. Situated between each vertebral body is an intervertebral disc that cushions and dampens compressive forces exerted upon the spinal column. A vertebral canal containing the spinal cord is located behind the vertebral bodies.

There are many types of spinal column disorders including scoliosis (abnormal lateral curvature of the spine), excess kyphosis (abnormal forward curvature of the spine), excess lordosis (abnormal backward curvature of the spine), spondylolisthesis (forward displacement of one vertebra over another), and other disorders caused by abnormalities, disease or trauma, such as ruptured or slipped discs, degenerative disc disease, fractured vertebrae, and the like. Patients that suffer from such conditions usually experience extreme and debilitating pain as well as diminished nerve function.

A noteworthy trend in the medical community is the move away from performing surgery via traditional "open" techniques in favor of minimally invasive or minimal access techniques. Open surgical techniques are generally undesirable in that they typically require large incisions and high amounts of tissue displacement to gain access to the surgical target site, which produces concomitantly high amounts of pain, lengthened hospitalization (increasing health care costs), and high morbidity in the patient population. Less-invasive surgical techniques are gaining favor due to the fact that they involve accessing the surgical target site via incisions of substantially smaller size with greatly reduced tissue displacement requirements. This, in turn, reduces the pain, morbidity, and cost associated with such procedures. One such minimally invasive approach, a lateral trans-psoas approach to the spine, developed by NuVasive^{®}, Inc., San Diego, CA (XLIF^{®}) has demonstrated great success in reducing patient morbidity, shortening the duration of hospitalization, and speeding recovery time if it is employed.

To create the lateral access corridor to the lumbar spine, the patient is positioned on his or her spine and a surgical access system is advanced through an incision, into the retroperitoneal space, and then through the psoas muscle until the target spinal site (for example, a disc space between a pair of adjacent vertebral bodies) is reached. The surgical access system may include a sequential dilation assembly of increasing diameter and a tissue retraction assembly. The sequential dilation assembly is advanced to the surgical target site and the retractor assembly is then advanced to the target site over the sequential dilation system. Stimulating electrodes may be provided on the distal tip of one or more different components of the surgical access system. Neurophysiologic monitoring may be performed while advancing one or more components of the dilation and retraction assemblies to the target site to detect the presence of, and thereby avoid, nerves lying in the trans-psoas path to the target site.

Once the retractor assembly has been docked at a target site however, a nerve may become compromised due to a variety of factors including, but not limited to, compression of the nerve due to inadvertent contact with the retractor blade and patient positioning on the surgical table. Stimulating within the surgical site provides information regarding the health and status of nearby nerves within the surgical site during maintenance of the lateral access corridor. However, the portion of a nerve that is compressed or otherwise affected may not lie within the surgical site such that information regarding the health and status of a greater portion of the motor neural pathway is desirable. Other methods of stimulating the motor neural pathway (e.g., transcranial electric motor evoked potential monitoring (MEP)) use multi-pulse trains of stimuli with high stimulus intensities and depolarize all nerves along the corticospinal pathway and result in muscle activity of many muscles of the head, upper extremities, torso, and lower extremities. This whole-body stimulation can sometimes lead to large amounts of patient movement during the procedure. It is generally preferable to conduct neurophysiologic monitoring with the least amount of stimulation intensity (and patient movement) as possible. MEP monitoring is also disadvantageous for monitoring the lower motor neural pathway in that requires the use of total intravenous anesthesia (TIVA). TIVA requires close monitoring and is also more expensive than other anesthetic regimens. Furthermore, information regarding each specific nerve root is also desirable because it provides specific information regarding the health and/or status of each nerve root comprising the lumbar plexus. Therefore, a need exists for systems and methods of performing neurophysiologic monitoring on a greater portion of the motor neural pathway, lower amounts of stimulation intensity, shorter pulses, well-received anesthetic requirements, greater specificity of the at-risk nerve roots and earlier indications of potential post-operative complications such that mitigating actions may be taken.

### SUMMARY OF THE INVENTION

The present invention provides a non-transitory computer-readable medium for monitoring the health of the lower motor neural pathway during a spinal procedure, as set out in claim 1.

The present disclosure includes systems and methods to evaluate the health and status of the lower motor neural pathway before, during and after the establishment of an operative corridor through (or near) any of a variety of tissues having such neural structures which, if contacted or impinged, may otherwise result in neural impairment for the patient. It is expressly noted that, although described herein largely in terms of use in lateral lumbar spinal surgery, the system and methods of the present disclosure are suitable for use in any number of additional spinal surgeries including posterior, posterolateral, anterior, anterolateral lumbar spinal surgeries as well as thoracic and thoracolumbar spinal surgeries. Indeed, the present disclosure is suitable for use in any number of additional surgical procedures wherein tissue having significant neural structures must be passed through (or near) in order to establish an operative corridor.

According to another broad aspect, the present disclosure includes a control unit, a patient module, and a plurality of surgical accessories adapted to couple to the patient module. The control unit includes a power supply and is programmed to receive user commands, activate stimulation in a plurality of predetermined modes, process signal data according to defined algorithms, display received parameters and processed data, and monitor system status. The patient module is in communication with the control unit. The patient module is within the sterile field. The patient module includes signal conditioning circuitry, stimulator drive circuitry, and signal conditioning circuitry required to perform said stimulation in said predetermined modes. The patient module includes a processor programmed to perform a plurality of predetermined functions including at least two of neuromuscular pathway assessment, non-evoked monitoring, static pedicle integrity testing, dynamic pedicle integrity testing, nerve proximity detection, manual motor evoked potential monitoring, automatic motor evoked potential monitoring, transcutaneous nerve root testing, manual somatosensory evoked potential monitoring, automatic somatosensory evoked potential monitoring, and surgical correction planning and assessment.

According to still another broad aspect, the present disclosure includes a processing unit programmed to perform a plurality of predetermined functions using said instrument including at least two of neuromuscular pathway assessment, static pedicle integrity testing, dynamic pedicle integrity testing, nerve proximity detection, transcutaneous nerve root testing, non-evoked monitoring, motor evoked potential monitoring, somatosensory evoked potential monitoring, and surgical correction planning and assessment. The processing system has a pre-established profile for at least one of said predetermined functions so as to facilitate the initiation of said at least one predetermined function.

According to another broad aspect of the present disclosure, there is provided a method for performing transcutaneous, trans-abdominal stimulation of the lumbar motor neural pathways superior and inferior to a surgical target site.

### BRIEF DESCRIPTION OF THE DRAWINGS

Many advantages of the present invention will be apparent to those skilled in the art with a reading of this specification in conjunction with the attached drawings, wherein like reference numerals are applied to like elements and wherein:
Figure 1 is a block diagram of an example neurophysiologic monitoring system capable of conducting multiple nerve and spinal cord monitoring functions including but not necessarily limited to neuromuscular pathway, bone integrity, nerve detection, nerve pathology (evoked or free-run EMG), lower motor pathway, MEP, and SSEP assessments;
Fig. 2 is a perspective view showing examples of several components of the system of Fig. 1;
Fig. 3 is a graph illustrating a plot of a single pulse stimulation current signal capable of producing a neuromuscular response (EMG) of the type shown in Fig. 5;
Fig. 4 is a graph illustrating [a] plot of a stimulation current signal comprising a train of pulses capable of producing a neuromuscular response (EMG) of the type shown in Fig. 5;
Fig. 5 is a graph illustrating a plot of the neuromuscular response of a given myotome over time based on a stimulation signal (such as shown in either Fit. 4 of Fig. 5);
Fig. 6 is a perspective view of an example of a control unit forming part of the system of Fig. 1;
Figs. 7-9 are perspective, top, and side views, respectively, of an example of a patient module forming part of the system of Fig. 1;
Fig. 10 is a top view of an electrode harness forming part of the system of Fig. 1;
Figs. 11A-11C are side views of various examples of harness ports forming part of the system of Fig. 1;
Fig. 12 is a plan view of an example of a label affixed to an electrode connector forming part of the system of Fig. 1;
Figs. 13A-13B are top views of examples of electrode caps forming part of the system of Fig. 1;
Figs. 14A-14B are screenshots of an example of an electrode test screen forming part of the system of Fig. 1;
Fig. 15 is a perspective view of a stimulator forming part of the system of Fig. 1;
Fig. 16 is a perspective view of a second stimulator forming part of the system of Fig. 1;
Fig. 17 is a perspective view of the stimulators of Fig. 15 and 16 coupled together for use;
Figs. 18A-18B are perspective views third and fourth stimulators forming part of the system of Fig. 1;
Fig. 19 is a perspective view of the stimulators of Fig. 18A and 16 coupled together for use;
Figs. 20-21 are perspective views of an example of a secondary display forming part of the system of Fig. 1;
Fig. 21 is a first screenshot of an example of a TCNR Alert monitoring screen forming part of the neurophysiology system of Fig. 1;
Fig. 22 is a screenshot of an example of a profile screen forming part of the system of Fig. 1;
Fig. 23 is a screenshot of an example of a Twitch Test monitoring screen forming part of the system of Fig. 1;
Fig. 24 is a screenshot of an example of a Basic Stimulation EMG monitoring screen forming part of the system of Fig. 1;
Fig. 25 is a screenshot of an example of a Dynamic Stimulation EMG monitoring screen forming part of the system of Fig. 1;
Fig. 26 is a screenshot of an example of a Nerve Surveillance EMG monitoring screen forming part of the system of Fig. 1;
Fig. 27 is a screenshot of an example of a Manual MEP monitoring screen forming part of the system of Fig. 1;
Fig. 28 is a screenshot of an example of an Automatic MEP monitoring screen forming part of the system of Fig. 1;
Fig. 29 is a screenshot of an example of a TCNR setup screen forming part of the system of Fig. 1;
Fig. 30 is a first screenshot of an example of a TCNR Alert monitoring screen forming part of the system of Fig. 1;
Fig. 31 is a second screenshot of the TCNR Alert monitoring screen of Fig. 30;
Fig. 32 is a first screenshot of an example of a TCNR Threshold monitoring screen forming part of the system of Fig. 1;
Fig. 33 is a second screenshot of the TCNR Threshold monitoring screen of Fig. 32;
Fig. 34 is a third screenshot of the TCNR Threshold monitoring screen of Fig. 32;
Fig. 35 is a screenshot of an example of an SSEP Manual monitoring screen forming part of the system of Fig. 1;
Fig. 36 is a screenshot of an example of an SSEP Automatic monitoring screen form part of the system of Fig. 1;
Fig. 37 is a screenshot of an example of a first surgical correction planning and assessment screen forming part of the system of Fig. 1;
Fig. 38 is a screenshot of an example of second surgical correction planning and assessment screen forming part of the system of Fig. 1;
Figs. 39A-D are graphs illustrating the fundamental steps of a rapid current threshold-hunting algorithm;
Fig. 40 is a block diagram illustrating the steps of an initiation sequence for determining a relevant safety level prior to determining a precise threshold value according to an alternate threshold hunting algorithm of Figs. 39A-D;
Fig. 41 is a flowchart illustrating the method by which a multi-channel hunting algorithm determines whether to perform or omit a stimulation;
Figs. 42A-C are graphs illustrating use of the threshold hunting algorithm of Fig. 41 and further omitting stimulations when the likely result is already clear from previous data;
Fig. 43A is a flowchart illustrating the sequence employed by the algorithm to determine and monitor Ithresh;
Fig. 43B is a graph illustrating the confirmation step employed by the algorithm to determine whether Iₜhᵣₑₛh has changed from a previous determination; and
Fig. 44 is a flowchart illustrating the sequence of a rapid thresholding algorithm .

### DETAILED DESCRIPTION

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure. The systems disclosed herein boast a variety of inventive features and components that warrant patent protection, both individually and in combination. It is also expressly noted that, although described herein largely in terms of use in lateral surgery in the lumbar spine, the system and methods of the present disclosure may also be employed in any number of other spine surgery procedures including posterior, poster-lateral, anterior, and anterolateral lumbar, thoracic, and/or cervical spine procedures, all without departing from the present disclosure. The systems and methods described herein boast a variety of inventive features and components that warrant patent protection, both individually and in combination.

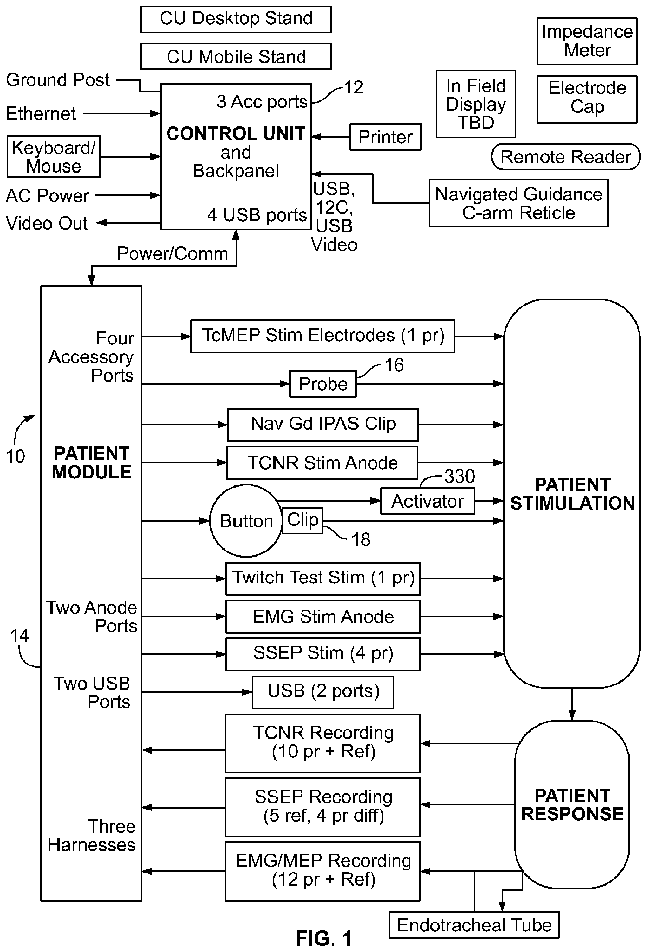

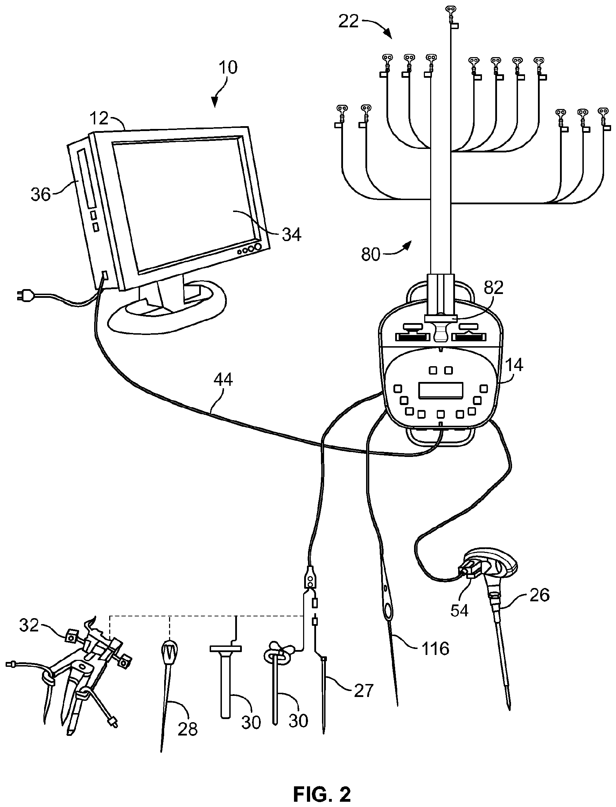

A neurophysiologic monitoring system 10 is described herein and is capable of performing a number of neurophysiological and/or guidance assessments at the direction of the surgeon (and/or other members of the surgical team). By way of example only, Figs. 1-2 illustrate the basic components of the system 10. The system comprises a control unit 12 (including a main display 34 preferably equipped with a graphical user interface (GUI) and a processing unit 36 that collectively contain the essential processing capabilities for controlling the system 10), a patient module 14, a stimulation accessory (e.g. a stimulation probe 16, stimulation clip 18 for connection to various surgical instruments, an inline stimulation hub 20, and stimulation electrodes 22), and a plurality of recording electrodes 24 for detecting electrical potentials.

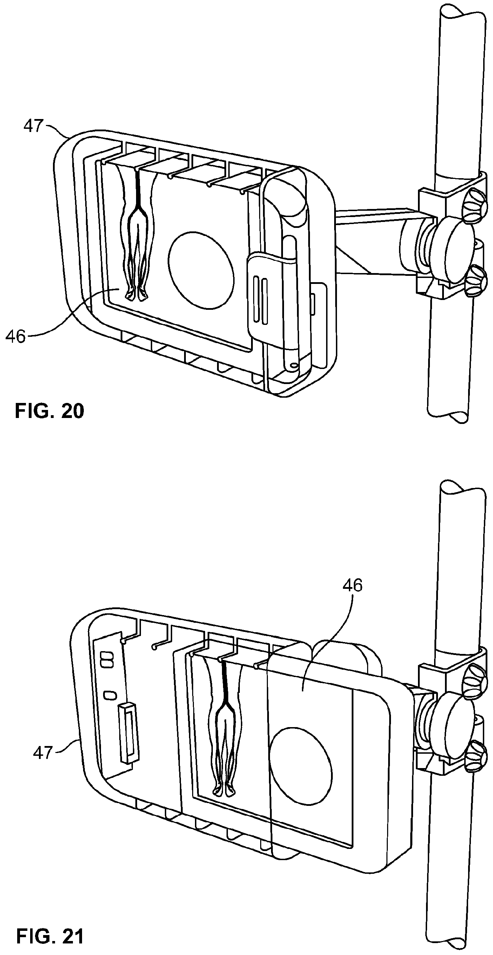

The stimulation accessories may be in the form of various prove devices that are themselves inserted into the stimulation site, clips that attach and deliver stimulation signals to standard instruments that are used at various times throughout a procedure...and surface electrodes. The stimulation clip 18 may be used to connect any of a variety of surgical instruments to the system 10, including, but not necessarily limited to a pedicle access needle 26, k-wire 27, tap 28, dilator(s) 30, tissue retractor 32, etc. One or more secondary feedback devices (e.g. secondary display 46 in Fig. 20-21) may also be provided for additional expression of output to a user and/or receiving input from the user.

The system 10 may be configured to execute any of the functional modes including, but not necessarily limited to, neuromuscular pathway assessment ("Twitch Test"), non-evoked monitoring ("Free-run EMG"), static pedicle integrity testing ("Basic Stimulated EMG"), dynamic pedicle integrity testing ("Dynamic Stimulated EMG"), nerve proximity detection ("XLIF^{®}"), motor evoked potential monitoring ("MEP Manual" and "MEP Automatic"), transcutaneous nerve root testing ("TCNR Alert" and "TCNR Threshold"), somatosensory evoked potential monitoring ("SSEP Manual" and "SSEP Automatic"), and surgical correction planning and assessment. The system 10 may also be configured for performance in any of the lumbar, thoracolumbar, and cervical regions of the spine.

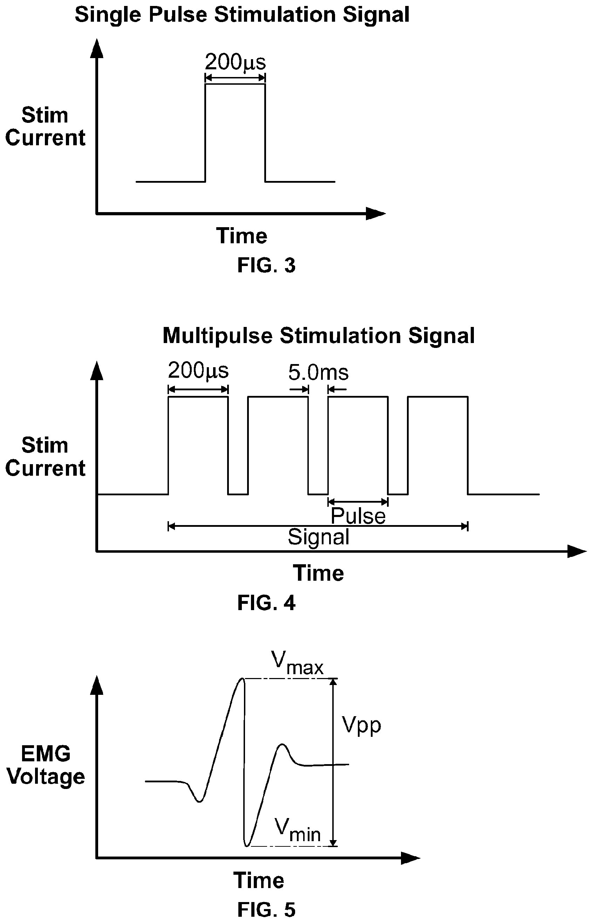

The basis for performing many of these functional modes (e.g. Twitch Test, Basic Stimulated EMG, Dynamic Stimulated EMG, XILF, MEP Manual, MEP Automatic, TCNR Alert, and TCNR Threshold) is the assessment of evoked responses of the various muscles myotomes monitored by the system 10 in relation to a stimulation signal transmitted by the system 10 (via patient module 14). The assessment of the evoked responses can be any suitable means of sensing physical motion of a muscle, for example via mechanomyography (MMG) which entails using an accelerometer or other similar device for detecting mechanical movement of a muscle or via electromyography (EMG) which is described in detail herein. This is illustrated in Figs. 3-5, wherein Fig. 5 illustrates the resulting EMG waveform of a monitored myotome in response to one of the example stimulation signals represented in Fig. 3 and Fig. 4. The EMG responses provide a quantitative measure of the nerve depolarization caused by the electrical stimulus. One way to characterize the EMG response is by a peak-to-peak voltage of V_{pf},^{,}Vₘₐₓ-Vₘₕᵢ, as shown in Fig. 5. Nerve tissues have characteristic threshold current levels (thᵣₑₛh) at which they will depolarize and result in a detectable muscle activity. Below this threshold current level, a stimulation signal will not evoke a significant EMG response. A significant EMG response may be defined as having a vₚₚ of approximately 100uV. Thus, the lowest stimulation current necessary to evoke an EMG response of the threshold voltage (Vₜhᵣₑₛh), 100uV in this example, may be called hᵣₑₛh. The greater the degree of electrical communication between a stimulation signal and a nerve, the lower 'thresh will be. Conversely, the lower the degree of electrical communication between a stimulation signal and a nerve, the greater _{Mesh} will be. Thus determining Ith,,ₛh, and/or monitoring changes in 'thᵣₑₛh over time, may provide valuable information when nerve tissues are at risk during a surgical procedure, as will be discussed in more detail below. By way of example, an excessively high Ithresh or an increase over a previous measurement during MEP testing may indicate a problem in the spinal cord or other portion of the motor pathway inhibiting transmission (communication) of the stimulation signal to the nerve. Meanwhile, during the Basic Stimulated EMG or Dynamic Stimulated EMG modes and the XLIF mode, a low 'thresh value may indicate a breach in the pedicle allowing the electrical signal to transmit through the pedicle, or the close proximity of a nerve to the stimulation source, respectively. Armed with the useful information conveyed by thresh, the surgeon may detect a problem or potential problem early and then act to avoid and/or mitigate the problem. The neurophysiology system 10 may quickly and accurately determine _{Ithresh} under the direction and operation of the surgeon (if desired) and convey the useful information Iₜhᵣₑₛh contains in a simple and easily comprehensible manner for interpretation by the surgeon.

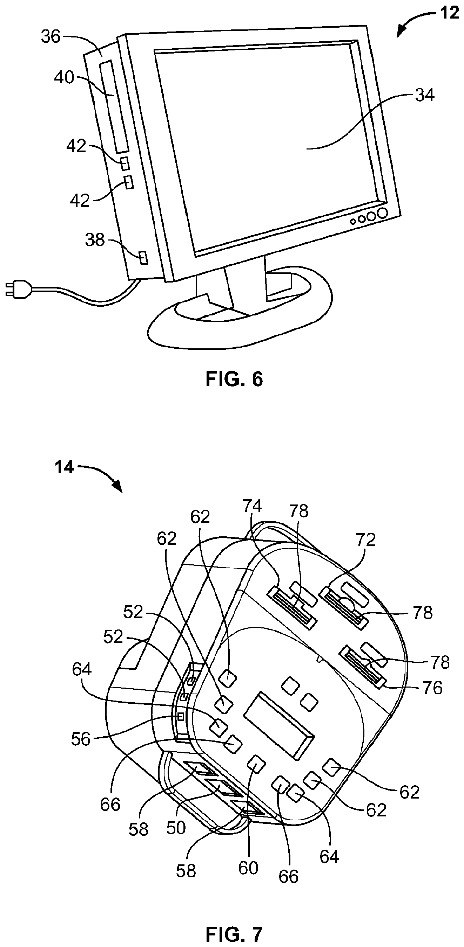

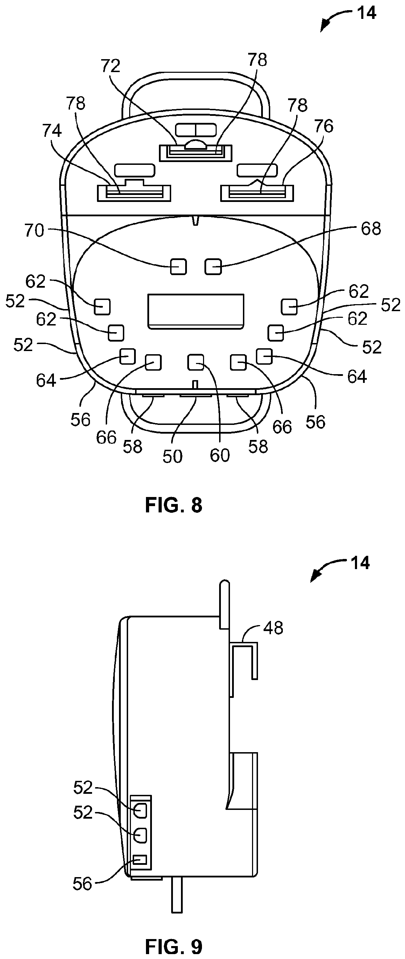

Before further addressing the various functional modes of the surgical system 10, the hardware components and features of the system 10 will be describe in further detail. The control unit 12 of the system 10, illustrated by way of example only in Fig. 6, includes a main display 34 and a processing unit 36, which collectively contain the essential processing capabilities for controlling the system 10. The main display 34 is preferably equipped with a graphical user interface (GUI) capable of graphically communicating information to the user and receiving instructions from the user. The processing unit 36 contains computer hardware and software that commands the stimulation source (e.g. patient module 14, Figs. 7-9), receives digital and/or analog signals and other information from the patient module 14, processes EMG and SSEP response signals, and displays the processed data to the user via the display 34. The primary functions of the software within the control unit 12 include receiving user commands via the touch screen main display 34, activating stimulation in the appropriate mode (Twitch Test, Basic Stimulated EMG, Dynamic Stimulated EMG, XLIF, MEP Manual, MEP Automatic, TCNR Alert, TCNR Threshold, SSEP Manual, and SSEP Automatic), processing signal data according to defined algorithms, displaying received parameters and processed data, and monitoring system status. The main display 34 may comprise a 15" LCD display equipped with suitable touch screen technology and the processing unit 36 may comprise a 2GHz. The processing unit 36 shown in Fig. 6 further includes a powered USB port 38 for connection to the patient module 14, a media drive 40 (e.g. CD, CD-RW, DVD, DVD-RW, etc...), a network port, wireless network card, and a plurality of additional ports 42 (e.g. USB, IEEE 1394, infrared, etc...) for attaching additional accessories, such as for example only, navigated guidance sensors, auxiliary stimulation anodes, and external devices (e.g. printer, keyboard, mouse, etc...). Preferably, during use the control unit 12 sits near the surgical table but outside the surgical field, such as for example, on a table top or a mobile stand. It will be appreciated, however, that if properly draped and protected, the control unit 12 may be located within the surgical (sterile) field.

The patient module 14, shown by way of example only in Figs. 4-6, is communicatively linked to the control unit 12. The patient module 14 is communicatively linked with and receives power from the control unit 12 via a USB data cable 44. However, it will be appreciated that the patient module 14 may be supplied with its own power source and other known data cables, as well as wireless technology, may be utilized to establish communication between the patient module 14 and control unit 12. The patient module 14 contains a digital communications interface to communicate with the control unit 12, as well as the electrical connections to all recording and stimulation electrodes, signal conditioning circuitry, stimulator drive and steering circuitry, and signal conditioning circuitry required to perform all of the functional modes of the system 10, including but not necessarily limited to Twitch Test, Free-run EMG, Basic Stimulated EMG, Dynamic Stimulated EMG, XLIF, MEP Manual and MEP Automatic, TCNR Alert, TCNR Threshold, SSEP Manual, and SSEP Automatic. In one example, the patient module 14 includes thirty-two recording channels and eleven stimulation channels. A display (e.g. an LCD screen) may be provided on the face of the patient module 14, and may be utilized for showing simple status readouts (for example, results of a power on test, the electrode harnesses attached, and impedance data, etc...) or more procedure related data (for example, a stimulation threshold result, current stimulation level, selected function, etc...). The patient module 14 may be positioned near the patient in the sterile field during surgery. By way of example, the patient module 14 may be attached to bed rail with the aid of a hook 48 attached to, or forming a part of, the patient module 14 casing.

With reference to Figs. 7-9, patient module 14 comprises a multitude of ports and indicators for connecting and verifying connections between the patient module 14 and other system components. A control unit port 50 is provided for data and power communication with the control unit 12, via USB data cable 44 as previously described. There are four accessory ports 52 provided for connecting up to the same number of surgical accessories, including, but not necessarily limited to, stimulation probe 16, stimulation clip 18, inline stimulation hub 20, and navigated guidance sensor (or tilt sensor) 54. The accessory ports 52 include a stimulation cathode and transmit digital communication signals, tri-color LED drive signals, button status signals, identification signals, and power between the patient module 14 and the attached accessory. A pair of anode ports 56, preferably comprising 2 wire DIN connectors, may be used to attach auxiliary stimulation anodes should it become desirable or necessary to do so during a procedure. A pair of USB ports 58 are connected as a USB hub to the control unit 12 and may be used to make any number of connections, such as for example only, a portable storage drive.

As soon as a device is plugged into any one of ports 50, 52, 56, or 58, the system 10 automatically performs a circuit continuity check to ensure the associated device will work properly. Each device forms a separate closed circuit with the patient module such that the devices may be checked independent of each other. If one device is not working properly the device may be identified individually while the remaining devices continue indicate their valid status. An indicator LED is provided for each port to convey the results of the continuity check to the user. Thus, according to the example of Figs. 7-9, the patient module 14 includes one control unit indicator 60, four accessory indicators 62, two anode indicators 64, and two USB indicators 66. If the system detects an incomplete circuit during the continuity check, the appropriate indicator will turn red alerting the user that the device might not work properly. On the other hand, if a complete circuit is detected, the indicator will appear green signifying that the device should work as desired. Additional indicator LEDs are provided to indicate the status of the system and the MEP stimulation. The system indicator 68 will appear green when the system is ready and red when the system is not ready. The MEP stim indicator 70 lights up when the patient module is ready to deliver and MEP stimulation signal. The MEP stim indicator 68 appears yellow to indicate a ready status.

To connect the array of recording electrodes 24 and stimulation electrodes 22 utilized by the system 10, the patient module 14 also includes a plurality of electrode harness ports. The patient module 14 includes an EMG/MEP harness port 72, SSEP harness port 74, an Auxiliary harness port 76 (for expansion and/or custom harnesses; e.g, a TCNR harness). Each harness port 72, 74, and 76 includes a shaped socket 78 that corresponds to a matching shaped connector 82 on the appropriate electrode harness 80. In addition, the system 10 may preferably employ a color code system wherein each modality (e.g. EMG, EMG/MEP, and SSEP) has a unique color associated with it. By way of example only and as shown herein, EMG monitoring (including, screw tests, detection, and nerve retractor) may be-associated with the color green, MEP monitoring with the color blue, and SSEP monitoring may be associated with the color orange. Thus, each harness port 72, 74, 76 is marked with the appropriate color which will also correspond to the appropriate harness 80. Utilizing the combination of the dedicated color code and the shaped socket connector interface simplifies the setup of the system, reduces errors, and can greatly minimize the amount of pre-operative preparation necessary. The patient module 14, and especially the configuration of quantity and layout of the various ports and indicators, has been described according to one example . It should be appreciated, however, that the patient module 14 could be configured with any number of different arrangements without departing from the scope of the invention.

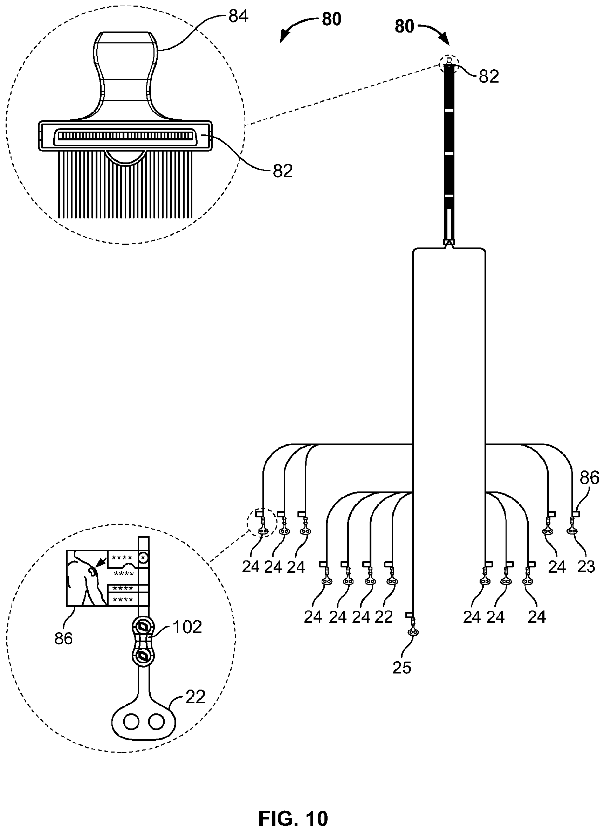

As mentioned above, to simplify setup of the system 10, all of the recording electrodes 24 and stimulation electrodes 22 that are required to perform one of the various functional modes (including a common electrode 23 providing a ground reference to pre-amplifiers in the patient module 14, and an anode electrode 25 providing a return path for the stimulation current) are bundled together and provided in single electrode harness 80, as illustrated, by way of example only, in Fig. 10. Depending on the desired function or functions to be used during a particular procedure, different groupings of recoding electrodes 24 and stimulation electrodes 22 may be required. By way of example, the SSEP function requires more stimulating electrodes 22 than either the EMG or MEP functions, but also requires fewer recording electrodes than either of the EMG and MEP functions. To account for the differing electrode needs of the various functional modes, the system 10 may employ different harnesses 80 tailored for the desired modes. Three different electrode harnesses 80 may be provided for use with the system 10, an EMG harness, an EMG/MEP harness, and an SSEP harness.

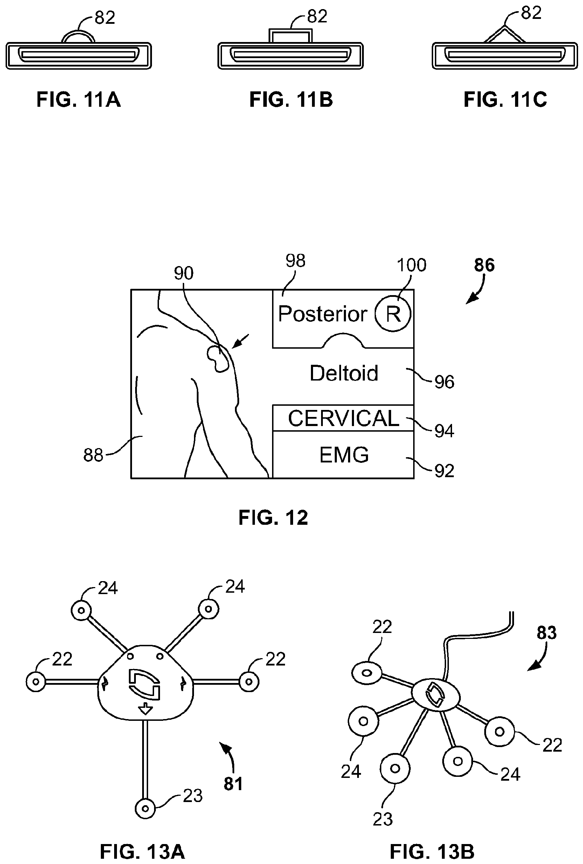

At one end of the harness 80 is the shaped connector 82. As described above, the shaped connector 82 interfaces with the shaped socket 72, 74, or 76 (depending on the functions harness 80 is provided for). Each harness 80 utilizes a shaped connector 82 that corresponds to the appropriate shaped socket 72, 74, 76 on the patient module 14. If the shapes of the socket and connector do not match the harness 80, connection to the patient module 14 cannot be established. The EMG and the EMG/MEP harnesses both plug into the EMG/MEP harness port 72 and thus they both utilize the same shaped connector 82. By way of example only, Figs. 11A-11C illustrate the various shape profiles used by the different harness ports 72, 74, 76 and connectors 82. Fig. 11A illustrates the half circular shape associated with the EMG and EMG/MEP harness and port 72. Fig. 11B illustrates the rectangular shape utilized by the SSEP harness and port 74. Finally, Fig. 11C illustrates the triangular shape utilized by the Auxiliary harness and port 76. Each harness connector 82 includes a digital identification signal that identifies the type of harness 80 to the patient module 14. At the opposite end of the electrode harness 80 are a plurality of electrode connectors 102 linked to the harness connector 82 via a wire lead. Using the electrode connector 102, any of a variety of known electrodes may be used, such as by way of example only, surface dry gel electrodes, surface wet gel electrodes, and needle electrodes.

To facilitate easy placement of scalp electrodes used during MEP and SSEP modes, an electrode cap 81, depicted by way of example only in Fig. 13A may be used. The electrode cap 81 includes two recording electrodes 23 for SSEP monitoring, two stimulation electrodes 22 for MEP stimulation delivery, and an anode 23. Graphic indicators may be used on the electrode cap 81 to delineate the different electrodes. By way of example, lightning bolts may be used to indicate a stimulation electrode, a circle within a circle may be used to indicate recording electrodes, and a stepped arrow may be used to indicate the anode electrode. The anode electrode wire is colored white to further distinguish it from the other electrodes and is significantly longer that the other electrode wires to allow placement of the anode electrode on the patient's shoulder. The shape of the electrode cap 81 may also be designed to simplify placement. By way of example only, the cap 81 has a pointed end that may point directly toward the patient's nose when the cap 81 is centered on the head in the right orientation. A single wire may connect the electrode cap 81 to the patient module 14 or electrode harness 80, thereby decreasing the wire population around the upper regions of the patient. Alternatively, the cap 81 may be equipped with a power supply and a wireless antenna for communicating with the system 10. Fig. 13B illustrates another example of an electrode cap 83 similar to cap 81. Rather than using graphic indicators to differentiate the electrodes, colored wires may be employed. By way of example, the stimulation electrodes 22 are colored yellow, the recording electrodes 24 are gray, and the anode electrode 23 is white. The anode electrode is seen here configured for placement on the patient's forehead. Alternatively, the electrode cap (not shown) may comprise a strap or set of straps configured to be worn on the head of the patient. The appropriate scalp recording and stimulation sites may be indicated on the straps. By way of example, the electrode cap may be imbued with holes overlying each of the scalp recording sites (for SSEP) and scalp stimulation sites (for MEP). According to a further example, the border around each hole may be color coded to match the color of an electrode lead wire designated for that site. In this instance, the recording and stimulation electrodes designated for the scalp are preferably one of a needle electrode and a corkscrew electrode that can be placed in the scalp through the holes in the cap.

As will be explained in greater detail below, the electrodes of different sizes and configurations may be preferable for the TCNR mode than for MEP, EMG, and SSEP modes. According to some implementations, the posterior cathode is a single use cathode electrode that has a circular shape (radially symmetric) to simplify positioning (superficially on the dorsal midline, approximately over the conus medullaris at the L1-L2 spinal level). The full contact surface is a conductive adhesive hydrogel to eliminate the need for skin prep. The connecting lead is made of insulated radiolucent carbon wire to avoid obscuring fluoroscopic images. The terminating 1.5mm female DIN connector is color coded purple to maintain correct polarity corresponding with the mating harness connector. The anterior anode is a single use anode electrode has a square shape (radially symmetric) with a relatively large surface area to simplify positioning (superficially on the abdominal midline below the umbilicus) and increase the positioning location tolerance. The full contact surface is a conductive adhesive hydrogel to eliminate the need for skin prep. The connecting lead is made of insulated radiolucent carbon wire to avoid obscuring fluoroscopic images. The terminating 1.5mm female DIN connector is color coded yellow maintain correct polarity corresponding with the mating harness connector.

In addition to or instead of color coding the electrode lead wires to designated intended placement, the end of each wire lead next to the electrode connector 102 may be tagged with a label 86 that shows or describes the proper positioning of the electrode on the patient. The label 86 preferably demonstrates proper electrode placement graphically and textually. As shown in Fig. 12, the label may include a graphic image showing the relevant body portion 88 and the precise electrode position 90. Textually, the label 86 may indicate the side 100 and muscle (or anatomic location) 96 for placement, the function of the electrode (e.g. stimulation, recording channel, anode, and reference - not shown), the patient surface (e.g. anterior or posterior), the spinal region 94, and the type of monitoring 92 (e.g. EMG, MEP, SSEP, by way of example, only). According to one example, the electrode harnesses 80 are designed such that the various electrodes may be positioned about the patient (and preferably labeled accordingly) as described in Table 1 for Lumbar EMG, Table 2 for Cervical EMG, Table 3 for Lumbar/Thoracolumbar EMG and MEP, Table 4 for Cervical EMG and MEP, Table 5 for TCNR, and Table 6 for SSEP:

**Table 1: Lumbar EMG**

| **Electrode Type** | **Electrode Placement** | **Spinal Level** |
|---|---|---|
| Ground | Upper Outer Thigh | |
| Anode | Latissimus Dorsi | |
| Stimulation | Knee | |
| Recording | Left Tibialis Anterior | IA, L5 |
| Recording | Left Gastroc. Medialis | Si, S2 |
| Recording | Left Vastus Medialis | L2, L3, IA |
| Recording | Left Biceps Femoris | L5, Si, S2 |
| Recording | Right Biceps Femoris | L5, Si, S2 |
| Recording | Right Vastus Medialis | L2, L3, IA |
| Recording | Right Gastroc. Medialis | Si, S2 |
| Recording | Right Tibialis Anterior | IA, L5 |

**Table 2: Cervical EMG**

| **Electrode Type** | **Electrode Placement** | **Spinal Level** |
|---|---|---|
| Ground | Shoulder | |
| Anode | Mastoid | |
| Stimulation | Inside Elbow | - |
| Recording | Left Triceps | C7, C8 |
| Recording | Left Flexor Carpi Radial i s | C6, C7, C8 |
| Recording | Left Deltoid | C5, C6 |
| Recording | Left Trapezius | C3, C4 |
| Recording | Left Vocal Cord | RLN |
| Recording | Right Vocal Cord | RLN |
| Recording | Right Trapezius | C3, C4 |
| Recording | Right Deltoid | C5, C6 |
| Recording | Right Flexor Carpi Radialis | C6, C7, C8 |
| Recording | Right Triceps | C7, C8 |

**Table 3: Lumbar/Thoracolumbar EMG + MEP**

| **Electrode Type** | **Electrode Placement** | **Spinal Level** |
|---|---|---|
| Ground | Upper Outer Thigh | - |
| Anode | Latissimus Dorsi | - |
| Stimulation | Knee | - |
| Recording | Left Tibialis Anterior | L4, L5 |
| Recording | Left Gastroc. Medialis | S1, S2 |
| Recording | Left Vastus Medialis | L2, L3, L4 |
| Recording | Left Biceps Femoris | L5, S1,S2 |
| Recording | Left APB-ADM | C6, C7, C8, T1 |
| Recording | Right APB-ADM | C6, C7, C8, T1 |
| Recording | Right Biceps Femoris | L5, S1, S2 |
| Recording | Right Vastus Medialis | L2, L3, L4 |
| Recording | Right Gastroc. Medialis | S1, S2 |
| Recording | Right Tibialis Anterior | L4, L5 |

**Table 4: Cervical EMG + MEP**

| **Electrode Type** | **Electrode Placement** | **Spinal Level** |
|---|---|---|
| Ground | Shoulder | - |
| Anode | Mastoid | - |
| Stimulation | Inside Elbow | - |
| Recording | Left Tibialis Anterior | L4, L5 |
| Recording | Left Flexor Carpi Radialis | C6, C7, C8 |
| Recording | Left Deltoid | C5, C6 |
| Recording | Left Trapezius | C3, C4 |
| Recording | Left APB-ADM | C6, C7, C8, T1 |
| Recording | Left Vocal Cord | RLN |
| Recording | Right Vocal Cord | RLN |
| Recording | Right APB-ADM | C6, C7, C8, T1 |
| Recording | Right Trapezius | C3, C4 |
| Recording | Right Deltoid | C5, C6 |
| Recording | Right Flexor Carpi Radialis | C6, C7, C8 |
| Recording | Right Tibialis Anterior | L4, L5 |

**Table 5: Transcutaneous Nerve Root Stimulation**

| **Electrode Type** | **Electrode Placement** | **Spinal Level** |
|---|---|---|
| Ground | Hip | - |
| Anode | Mid-back | - |
| Stimulation | L1-L2 cathode | - |
| Stimulation | Umbilicus anode | - |
| Recording | Left Adductor Magnus | L2, L3, L4 |
| Recording | Left Vastus Lateralis | L3, L4 |
| Recording | Left Tibialis Anterior | L4, L5 |
| Recording | Left Biceps Femoris | L5, S1, S2 |
| Recording | Right Adductor Magnus | L2, L3, L4 |
| Recording | Right Vastus Lateralis | L3, L4 |
| Recording | Right Vastus Medialis | L2, L3, L4 |
| Recording | Right Tibialis Anterior | L4, L5 |
| Recording | Right Biceps Femoris | L5, S1, S2 |

**Table 6: SSEP**

| **Electrode Type** | **Electrode Placement** | **Spinal Level** |
|---|---|---|
| Ground | Shoulder | - |
| Stimulation | Left Post Tibial Nerve | - |
| Stimulation | Left Ulnar Nerve | - |
| Stimulation | Right Post Tibial Nerve | - |
| Stimulation | Right Ulnar Nerve | - |
| Recording | Left Popliteal Fossa | - |
| Recording | Left Erb's Point | - |
| Recording | Left Scalp Cp3 | - |
| Recording | Right Popliteal Fossa | - |
| Recording | Right Erb's Point | - |
| Recording | Right Scalp Cp4 | - |
| Recording | Center Scalp Fpz | - |
| Recording | Center Scalp Cz | - |
| Recording | Center Cervical Spine | - |

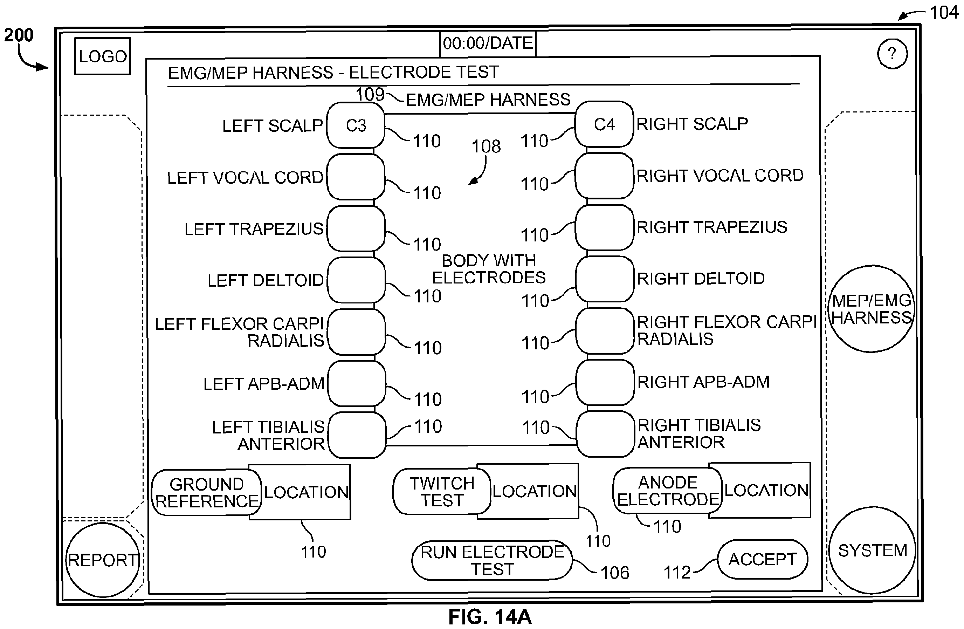

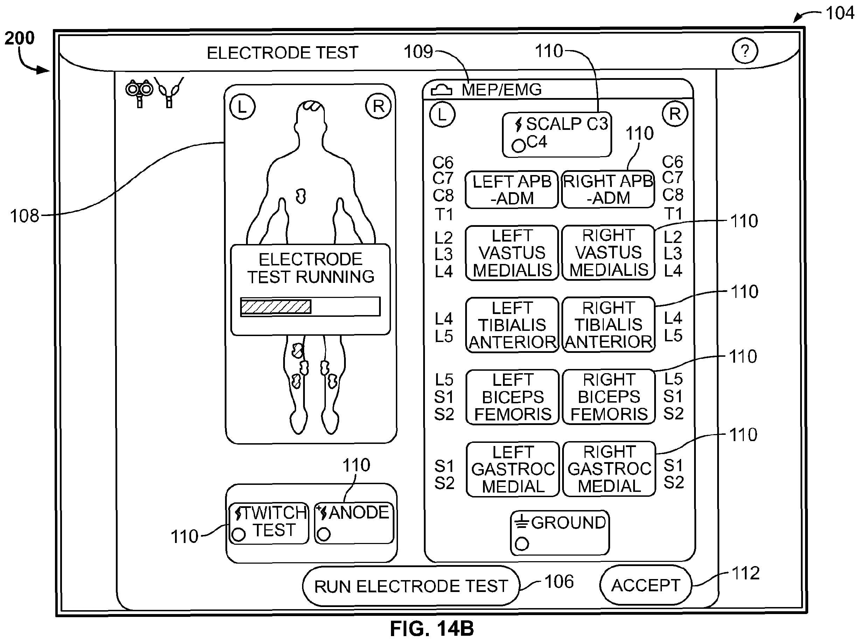

The patient module 14 is configured such that the system 10 may conduct an impedance test under the direction of the control unit 12 of all electrodes once the system is set up and the electrode harness is connected and applied to the patient. After choosing the appropriate spinal site upon program startup (described below), the user is directed to an electrode test. Figs. 14A-14B illustrate, by way of example only, a graphical implementation capturing the features of an electrode test[s] as implemented on an electrode test screen 104. The electrode test screen 104 includes a human figure depiction with positioned electrodes 108. A harness indicator 109 displays which harness is in use. For each electrode on the harness 80 in use there is a channel button 110. This includes the common 25 and anode 23 electrodes which are both independently checked for impedance. To accomplish this, the anode 23 and common 25 are both provided as dual electrodes. At least one of the anode leads on the anode electrode is reversible. During the impedance check, the reversible anode lead switches to a cathode such that the impedance between the leads can be measured. When the impedance test is complete, the reversible lead switches back to an anode. The channel button 110 may be labeled with the muscle or coverage area of the corresponding electrode. Selecting the channel button 110 will disable the channel.

Disabled channels will not be tested for impedance and they will not be monitored for responses or errors unless reactivated. Upon selection of a start button 106 ("Run Electrode Test"), the system tests each electrode individually to determine the impedance value. If the impedance is determined to be within acceptable limits, the channel button 110 and electrode depiction on the human figure 108 turn green. If the impedance value for any electrode is not determined to be acceptable, the associated channel button 110 and electrode depiction turn red, alerting the user. Once the test is complete, selecting the "Accept" button 112 will open the main monitoring screen 200 of the system 10.

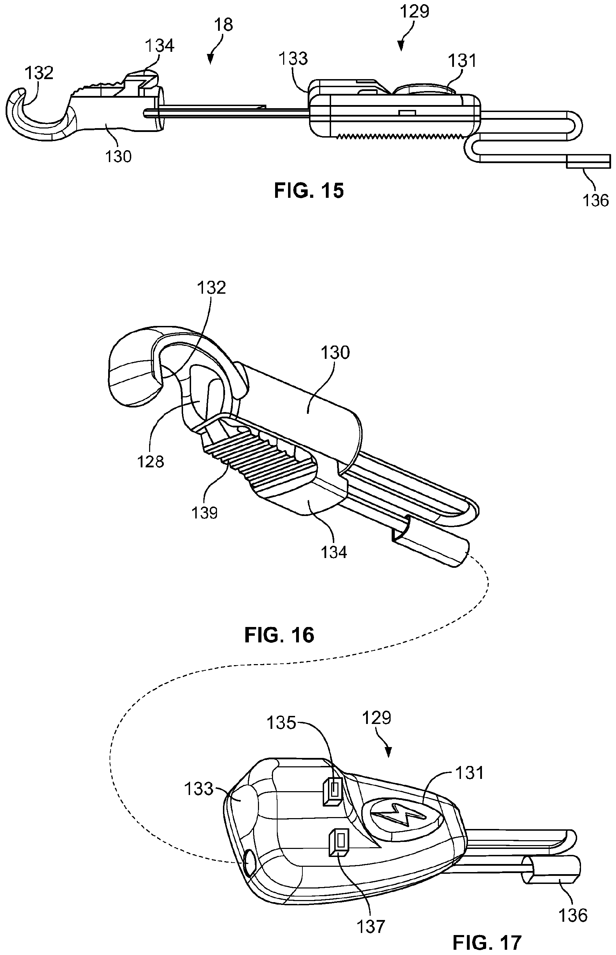

The system 10 may utilize various stimulation accessories to deliver stimulation signals to a stimulation target site such as over the patient's conus medullaris, a hole formed or being formed in a pedicle, and/or tissue surrounding an access corridor. Figs. 15-17 illustrate an example of a stimulation accessory in the form of a stimulation clip 18 that permits the system 10 to deliver stimulation signals through various surgical instruments already used during the surgical procedure. By way of example only, the coupling device 18 may connect the system 10 with instruments including, but not necessarily limited to, a pedicle access needle 26, a tap 28, dilator 30, tissue retractor 32, and k-wire 27. The stimulation clip 18 utilizes a spring-loaded plunger 128 to hold the surgical tool and transmit the stimulation signal thereto. The plunger 128 is composed of a conductive material such as metal. A nonconductive housing 130 partially encases the plunger 128 about its center. Extending from the housing 130 is an endplate 132 that hooks the surgical instrument. A spring (not shown) is disposed within the housing 130 such that in a natural or "closed" state, the plunger 128 is situated in close proximity to the endplate 132. Exerting a compressive force on the spring (such as by pulling on the thumb grip 134) causes a gap between the end plate 132 and the plunger 128 to widen to an "open" position (shown in Figs. 15-17 thereby allowing insertion of a surgical tool between the endplate 132 and plunger 128. Releasing the thumb grip 134 allows the spring to return to a "closed" position, causing the plunger 132 to move laterally back towards the endplate such that a force is exerted upon the surgical instrument and thereby holding it in place between the endplate 132 and the plunger 128. The clip 18 further includes a button module 129 containing an activation button 131 for initiating stimulation. The button module 129 is set apart from the body of the clip 18 and they are linked by an integrated wire. An accessory port 133 is located next to the button 131 on the button module 129, thus minimizing the number of wires connecting back to the patient module 14 and outside the sterile field. Clip 18 is equipped with three LEDs 135, 137, and 139. LED 135 is associated with the accessory port 133 and LED 137 is associated with the clip 18 to indicate which of the two is stimulating. The LEDs 137 and 137 may appear purple when stimulation is active. When a stimulation result is determined, the associated LED 135 or 137 may appear either red (if the result meets a predetermined potentially unsafe value), green (if the result meets a predetermined safe value), or yellow (if the result is in between the safe and potentially unsafe values). A third LED 139 is contained within the thumb grip 134, which will appear red, yellow, or green depending on the threshold result. The clip 18 connects to one of the accessory ports 62 on the patient module 14 via a connector 136. The connector 136 includes an identification signal that identifies it to the patient module.

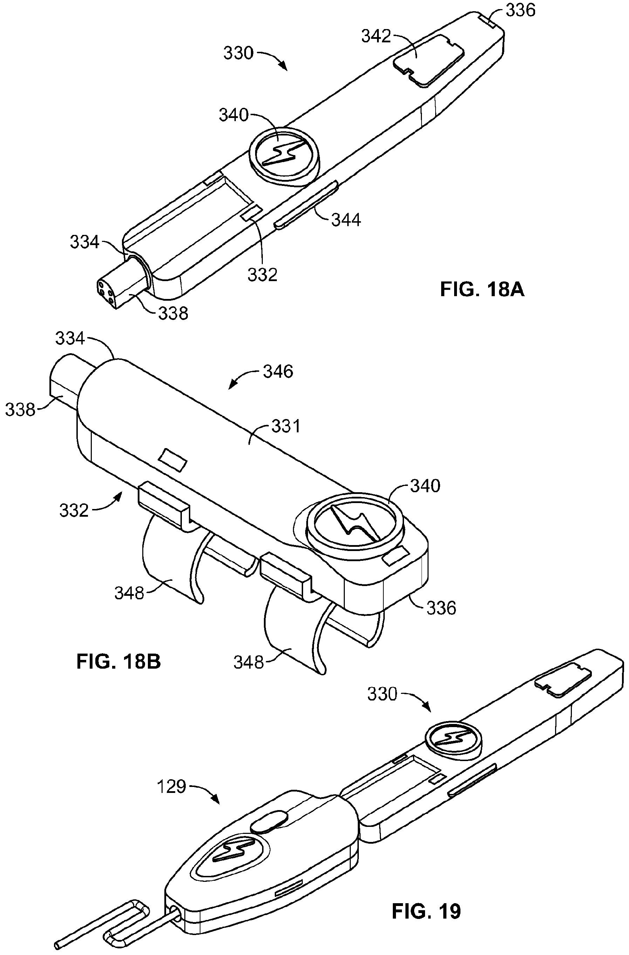

Fig. 18A illustrates a second example of a stimulation accessory in the form of an in-field activator 330. The activator 330 is preferably a single-use sterile device and may be designed [as] a stand-alone device designed to interface with the patient module 14 independently or may be part of an assembly as will be explained below. The activator 330 may plug into an accessory port 133 of the stimulation clip 18 of Figs. 15-17 above to provide user control and status indication of the TCNR mode from inside the sterile field during the surgical procedure. Fig. 19 shows the activator 330 plugged into accessory port 133 for in-field use. It is contemplated that the activator 330 is compatible with module 129 such that the activator 330 and clip 18 may be jointly connected to the system 10 via module 129 without the need for additional components, additional wires, and the like. The activator 330 includes a top 331, a bottom 332, a housing 333, a first end 334, a second end 336, a connection plug 338 emanating from said first end 334, a stimulation button 334 and a multicolor LED indicator 342 disposed between ends 334, 336. The connection plug 338 is sized and dimensioned to fit within the accessory port 133. Activator 330 may include one or more securing clips 348 (Fig. 18B) for securing the activator 330 to a surgical implement, such as, for example, an articulating arm or a retractor (not shown). The activator 330/stimulation clip 18 assembly may be connected to one of the accessory ports 62 on the patient module 14 via a connector 136. The connector 136 includes an identification signal that identifies it to the patient module. Upon connection of the activator 330, the system 10 software will enable TCNR mode,

placing the icon for the mode on the test selection tab 204, as well as enabling configuration and other settings within the profiles and set up screens.

The activator button 330 allows a user from the sterile field to both navigate to the TCNR mode and initiate trans-abdominal, transcutaneous stimulation in TCNR modes with a single button press. By way of example only, the activator 330 may allow a user to both access the TCNR mode and initiate TCNR stimulation with a single button press as will be described in greater detail below. The activation button 340 may be circular and is protected from unintended activation by a raised surrounding border. Finger grips 334 may be provided on the sides of the activator housing 332 near activation button 340 to stabilize the activator 330 during use.

The activator 342 is equipped with a multi-color LED indicator 342. In some examples (e.g. that shown in Fig. 18A), the stimulation button 340 and the LED indicator 342 are two distinct components. In other examples (e.g. that shown in Fig. 18B), the LED indicator 342 is integrated into the stimulation button 340. LED indicator 342 provides feedback to the user of various states of operation of the TCNR modality. By way of example only, LED indicator 342 will illuminate when the timer has expired, when the stimulator button 340 has been pressed and when the system 10 is delivering stimulation.

**Table 7:**

| | |
|---|---|
| LED Visual Feedback | Indication or Assessment |
| Off | Timer not elapsed, stimulation is inactive, or modality not selected |
| Blinking Green 1 Hz rat | Primary timer (default 5 min) has elapsed since last stimulation |
| Blinking Green 2 Hz rate | Secondary timer (default 10 min) has elapsed since last stimulation |
| Blinking Amber 4 Hz rate | Stimulation is active |
| Solid Green | Previous result- no significant change from baseline |
| Steady Yellow | Previous result- noteworthy change from baseline |
| Steady Red | Previous result- significant change from baseline |

As mentioned above, the system 10 may include a secondary display, such as for example only, the secondary display 46 illustrated in Figs. 20-21. The secondary display 46 may be configured to display some or all of the information provided on main display 34. The information displayed to the user on the secondary display 34 may include, but is not necessarily limited to, alpha-numeric and/or graphical information regarding any of the selected function modes (e.g. Twitch Test, Free-Run EMG, Basic Stimulated EMG, Dynamic Stimulated EMG, XLIF, MEP Manual, MEP Automatic, TCNR Alert, TCNR Threshold, SSEP Manual, SSEP Automatic, and surgical correction planning and assessment), attached accessories (e.g. stimulation probe 16, stimulation clip 18, tilt sensor 54), electrode harness or harnesses attached, impedance test results, myotome/EMG levels, stimulation levels, history reports, selected parameters, test results, etc...Secondary display 46 may be configured to receive user input in addition to its display function. The secondary display 46 can thus be used as an alternate control point for the system 10. The control unit 12 and secondary display 46 may be linked such that input may be received on from one display without changing the output shown on the other display. This would allow the surgeon to maintain focus on the patient and test results while still allowing other members of the OR staff to manipulate the system 10 for various purposes (e.g. inputting annotations, viewing history, etc...). The secondary display 46 may be battery powered. Advantageously, the secondary display 46 may be positioned inside the sterile field as well as outside the sterile field. For positioning within the sterile field a disposable sterile case 47 may be provided to house the display. Alternatively, the display 46 may be sterile bagged. Both the sterile case 47 and the secondary display 46 may be mounted to a pole, bed frame, light fixture, or other apparatus found near and/or in the surgical field. It is further contemplated that multiple secondary displays 46 may be linked to the control unit 12. This may effectively distribute neurophysiology information and control throughout the operating room. By way of example, a secondary display 46 may also be provided for the anesthesiologist. This may be particularly useful in providing the anesthesiologist with results from the Twitch Test and providing reminders about the use of paralytics, which may adversely affect the accuracy of the system 10. Wired or wireless technology may be utilized to link the secondary display 46 to the control unit 12.

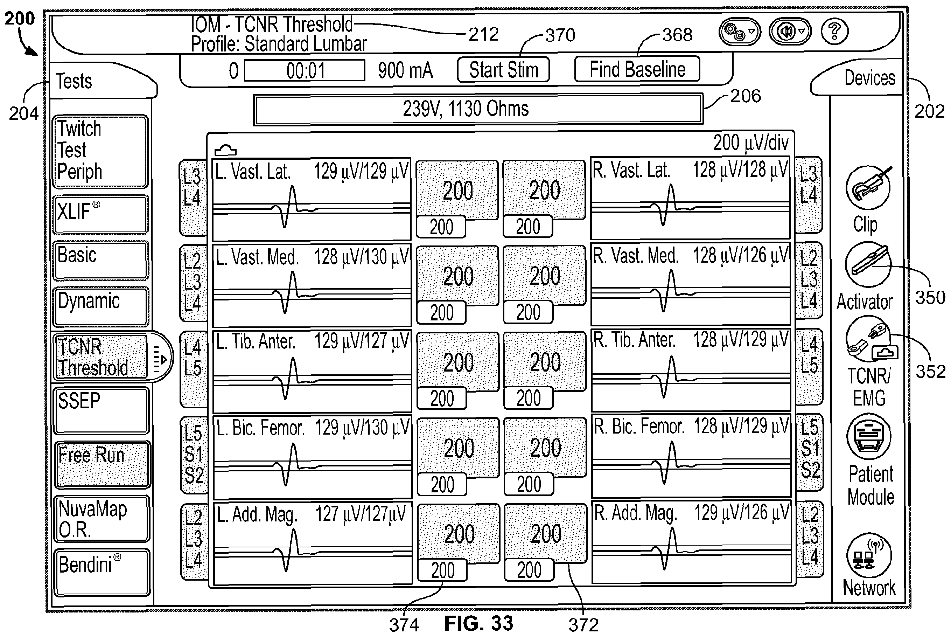

Having described an example of the system 10 and the hardware components that comprise it, the neurophysiological functionality and methodology of the system 10 will now be described in further detail. Various parameters and configurations of the system 10 may depend upon the target location (i.e. spinal region) of the surgical procedure and/or user preference. In one example, upon starting the system 10 the software will open to a startup screen, illustrated by way of example only, in Fig. 33. The startup screen includes a profile selection window 160 from which the user may select from one of the standard profiles (e.g. "Standard Cervical," "Standard Thoracolumbar," and "Standard Lumbar") or any custom profiles that have been previously saved to the system. Profiles may be arranged for selection, alphabetically, by spinal region, or by other suitable criteria. Profiles may be saved to the control unit hard drive or to a portable memory device, such as for example, a USB memory drive, or on a web server.

Selecting a profile configures the system 10 to the parameters assigned for the selected profile (standard or custom). The availability of different function modes may depend upon the profile selected. By way of example only, selecting the cervical and thoracolumbar spinal regions may automatically configure the options to allow selection of the Twitch Test, SSEP Manual, SSEP Automatic, Basic Stimulated EMG, Dynamic Stimulated EMG, XLIF, MEP Manual, MEP Automatic, Free-Run EMG modes, while selecting the lumbar region may automatically configure the options to allow selection of the Twitch Test, Basic, Difference, and Dynamic Stimulated EMG Tests, XLIF^{®}, and Nerve Retractor modes. Default parameters associated with the various function modes may also depend on the profile selected, for example, the characteristics of the stimulation signal delivered by the system 10 may vary depending on the profile. By way of example, the stimulation signal utilized for the Stimulated EMG modes may be configured differently when a lumbar profile is selected versus when one of a thoracolumbar profile and a cervical profile.

As previously described above, each of the hardware components includes an identification tag that allows the control unit 12 to determine which devices are hooked up and ready for operation. In one example, profiles may only be available for selection if the appropriate devices (e.g. proper electrode harness 80 and stimulation accessories) are connected and/or ready for operation. Alternatively, the software could bypass the startup screen and jump straight to one of the functional modes based on the accessories and/or harnesses it knows are plugged in. The ability to select a profile based on standard parameters, and especially on customized preferences, may save significant time at the beginning of a procedure and provides for monitoring availability right from the start. Moving on from the startup screen, the software advances directly to an electrode test screen and impedance tests, which are performed on every electrode as discussed above. When an acceptable impedance test has been completed, the system 10 is ready to begin monitoring and the software advances to a monitoring screen from which the neurophysiological monitoring functions of the system 10 are performed.

The information displayed on the monitoring screen may include, but is not necessarily limited to, alpha-numeric and/or graphical information regarding any of the functional modes (e.g. Twitch Test, Free-Run EMG, Basic Stimulated EMG, Dynamic Stimulated EMG, XLIF, MEP Manual, MEP Automatic, TCNR Alert, TCNR Threshold, SSEP Manual, SSEP Automatic, and surgical correction planning and assessment), attached accessories (e.g. stimulation probe 16, stimulation clip 18, tilt sensor 54), electrode harness or harnesses attached, impedance test results, myotome/EMG levels, stimulation levels, history reports, selected parameters, test results, etc... In one example, this information displayed on a main monitoring screen may include, but is not necessarily limited to, the following components as set forth in Table 8:

**Table 8**

| Screen Component | Description |
|---|---|
| Patient Image/ Electrode layout | An image of the human body or relevant portion thereof showing the electrode placement on the body, with labeled channel number tabs on each side (1-4 on the left and right). Left and right labels will show the patient orientation. The channel number tabs may be highlighted or colored depending on the specific function being performed. |
| Myotome & Level Names | A label to indicate the Myotome name and corresponding Spinal Level(s) associated with the channel of interest. |
| Test Menu | A hideable menu bar for selecting between the available functional modes. |
| Device Bar | A hideable bar displaying icons and/or names of devices connected to the patient module. |
| Display Area | Shows procedure-specific information including stimulation results. |
| Color Indication | Enhances stimulation results with a color display of green, yellow, or red corresponding to the relative safety level determined by the system. |
| Stimulation Bar | A graphical stimulation indicator depicting the present stimulation status (i.e. on or off and stimulation current level), as well as providing for starting and stopping stimulation |
| Event Bar | A hideable bar that shows the last up to a selected number of previous stimulation results, provides for annotation of results, and a chat dialogue box for communicating with remote participants. |
| EMG waveforms | EMG waveforms may be optionally displayed on screen along with the stimulation results. |

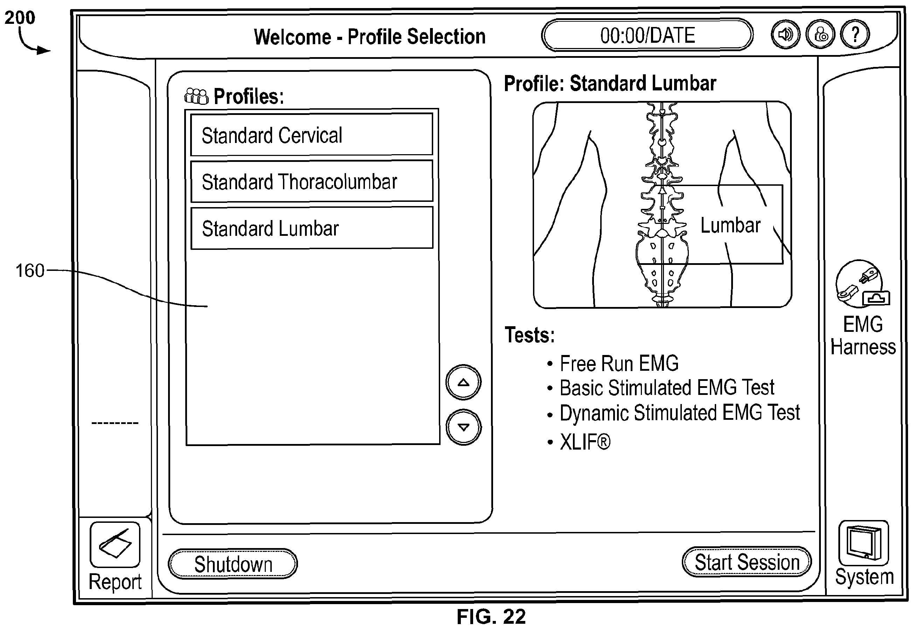

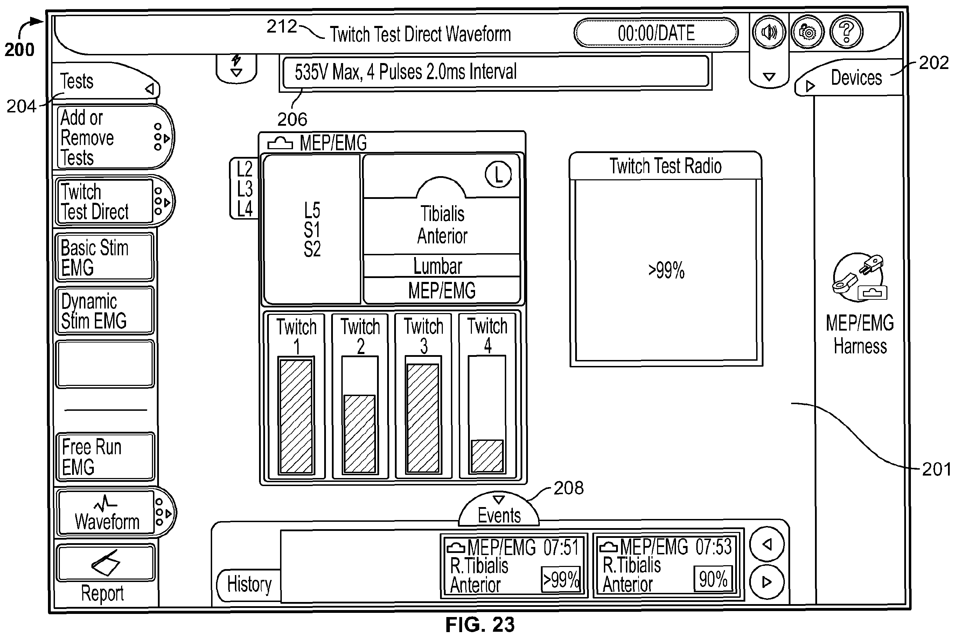

From a profile setting window 160, custom profiles can be created and saved. Beginning with one of the standard profiles, parameters may be altered by selecting one of the various buttons and making the changes until the desired parameters are set. By way of example only, profiles may be generated and saved for particular procedures (e.g. ACDF, XLIF, and decompression), particular individuals, and combinations thereof. Clicking on each button will display the parameter options specific to the selected button in a parameter window. The parameter options for the Test Selection Window are illustrated by way of example in Fig. 22. By way of example only, by selecting the Test Selection button, session tests may be added and viewing options may be changed. From within the test selection area, function specific parameters for all available test functions (based on site selection, available devices, etc...) may be accessed and set according to need. One option (not shown) that is available for multiple functions under the test selection button is the ability to select from three different viewing options. The user may choose to see results displayed in numeric form, on a body panel, and on a label that reflects the labels associated with each electrode, of any combination of the three. Figs. 23-38 illustrate examples of the test selection tab 204 for each of the test functions (e.g. Twitch Test, Basic Stimulated EMG, Dynamic Stimulated EMG, XLIF, TCNR Alert, TCNR Threshold, Free-Run, MEP Manual, MEP Automatic, SSEP Manual, SSEP Automatic). Profiles may be saved directly on the control unit 12, saved to a portable memory device, or uploaded onto a web-server.

The functions performed by the system 10 may include, but are not necessarily limited to, Twitch Test, Basic Stimulated EMG, Dynamic Stimulated EMG, XLIF^{®}, Nerve Retractor, TCNR Alert, TCNR Threshold, Free-run EMG, MEP Manual, MEP Automatic, SSEP Manual, SSEP Automatic, and surgical correction planning and assessment modes, all of which will be described below. The Twitch Test mode is designed to assess the neuromuscular pathway via the so-called "train-of-four-test" to ensure the neuromuscular pathway is free from muscle relaxants prior to performing neurophysiology-based testing, such as bone integrity (e.g. pedicle) testing, nerve detection, and nerve retraction. This is described in greater detail within PCT Patent App. No. PCT/US2005/036089, entitled "System and Methods for Assessing the Neuromuscular Pathway Prior to Nerve Testing," filed October 7, 2005. The Basic Stimulated EMG Dynamic Stimulated EMG tests are designed to assess the integrity of bone (e.g. pedicle) during all aspects of pilot hole formation (e.g., via an awl), pilot hole preparation (e.g. via a tap), and screw introduction (during and after). These modes are described in greater detail in PCT Patent App. No. PCT/US02/35047 entitled "System and Methods for Performing Percutaneous Pedicle Integrity Assessments," filed on October 30, 2002, and PCT Patent App. No. PCT/US2004/025550, entitled "System and Methods for Performing Dynamic Pedicle Integrity Assessments," filed on August 5, 2004. The XLIF mode is designed to detect the presence of nerves during the use of the various surgical access instruments of the system 10, including the pedicle access needle 26, k-wire 42, dilator 44, and retractor assembly 70. This mode is described in greater detail within PCT Patent App. No. PCT/US2002/22247, entitled "System and Methods for Determining Nerve Proximity, Direction, and Pathology During Surgery," filed on July 11, 2002. The Nerve Retractor mode is designed to assess the health or pathology of a nerve before, during, and after retraction of the nerve during a surgical procedure. This mode is described in greater detail within PCT Patent App. No. PCT/US2002/30617, entitled "System and Methods for Performing Surgical Procedures and Assessments," filed on Sept. 25, 2002

The MEP Manual and Automatic modes are designed to test the motor pathway to detect potential damage to the spinal cord by stimulating the motor cortex in the brain and recording the resulting EMG response of various muscles in the upper and lower extremities. The MEP Manual and Automatic modes are described in greater detail within PCT Patent App. No. PCT/US2006/003966, entitled "System and Methods for Performing Neurophysiologic Assessments During Spine Surgery," filed on February 2, 2006. The SSEP Manual and SSEP Automatic modes are designed to test the sensory pathway to detect potential damage to the spinal cord by stimulating peripheral nerves inferior to the target spinal level and recording the action potentials superior to the spinal level. The SSEP Manual and SSEP Automatic modes are described in greater detail within PCT Patent App. No. PCT/US2009/05650, entitled "Neurophysiologic Monitoring System and Related Methods," filed on October 15, 2009. The surgical correction planning and assessment modes are described in greater detail within PCT Patent Application No. PCT/US2014/059974, entitled "Systems for Planning, Performing, and Assessing Spinal Correction during Spine Surgery". These functions will be explained now in brief detail.

The system 10 performs neuromuscular pathway (NMP) assessments, via Twitch Test mode, by electrically stimulating a peripheral nerve (preferably the Peroneal Nerve for lumbar and thoracolumbar applications and the Median Nerve for cervical applications) via stimulation electrodes 22 contained in the applicable electrode harness and placed on the skin over the nerve or by direct stimulation of a spinal nerve using a surgical accessory such as the probe 116. Evoked responses from the muscles innervated by the stimulated nerve are detected and recorded, the results of which are analyzed and a relationship between at least two responses or a stimulation signal and a response is identified. The identified relationship provides an indication of the current state of the NMP. The identified relationship may include, but is not necessarily limited to, one or more of magnitude ratios between multiple evoked responses and the presence or absence of an evoked response relative to a given stimulation signal or signals. With reference to Fig. 23, details of the test indicating the state of the NMP and the relative safety of continuing on with nerve testing are conveyed to the surgeon via GUI display 34. On the monitoring screen 200 utilized by the various functions performed by the system 10, function specific data is displayed in a center result area 201. The results may be shown as a numeric value 210, a highlighted label corresponding to the electrode labels 86, or (in the case of twitch test only) a bar graph of the stimulation results. On one side of center result area 201 is a collapsible device menu 202. The device menu displays a graphic representation of each device connected to the patient module 14. Opposite the device menu 202 there is a collapsible test menu 204. The test menu 204 highlights each test that is available under the operable setup profile and may be used to navigate between functions. A collapsible stimulation bar 206 indicates the current stimulation status and provides start and stop stimulation buttons (not shown) to activate and control stimulation. The collapsible event bar 208 stores all the stimulation test results obtained throughout a procedure. Clicking on a particular event will open a note box and annotations may be entered and saved with the response for later inclusion in a procedure report. The event bar 208 also houses a chat box feature when the system 10 is connected to a remote monitoring system as described above. Within the result area 202 the twitch test specific results may be displayed.

It should be appreciated that while Fig. 23 depicts the monitoring screen 200 while the selected function is the Twitch Test, the features of monitoring screen 200 apply equally to all the functions. Result-specific data is displayed in a center result area 201. A large color saturated numeric value (not shown) is used to show the threshold result. Three different options are provided for showing the stimulation response level. First, the user can view the waveform. Second, a likeness of the color coded electrode harness label 86 may be shown on the display. Third, the color coded label 212 may be integrated with a body image. On one side of center result area 201 there is a collapsible device menu 202. The device menu displays a graphic representation of each device connected to the patient module 14. If a device is selected from the device menu 202, an impedance test may be initiated. Opposite the device menu 202 there is a collapsible test menu 204. The test menu 204 highlights each test that is available under the operable setup profile and may be used to navigate between functions. A collapsible stimulation bar 206 indicates the current stimulation status and provides start and stop stimulation buttons (not shown) to activate and control stimulation. The collapsible event bar 208 stores all the stimulation test results obtained throughout a procedure so that the user may review the entire case history from the monitoring screen. Clicking on a particular event will open a note box and annotations may be entered and saved with the response for later inclusion in a procedure report chronicling all nerve monitoring functions conducted during the procedure as well as the results of nerve monitoring. The report may be printed immediately from one or more printers located in the operating room or copied to any of a variety of memory devices known in the prior art, such as, by way of example only, a floppy disk, and/or USB memory stick. The system 10 may generate either a full report or a summary report depending on the particular needs of the user. The identifiers used to identify the surgical accessories to the patient module may also be encoded to identify their lot number or other identifying information. As soon as the accessory is identified, the lot number may be automatically added to the report. Alternatively, hand held scanners can be provided and linked to the control unit 12 or patient module 14. The accessory packaging may be scanned and again the information may go directly to the procedure report. The event bar 208 also houses a chat box feature when the system 10 is connected to a remote monitoring system to allow a user in the operating room to contemporaneously communicate with a person performing the associated neuromonitoring in a remote location.

The system 10 may also conduct free-run EMG monitoring while the system is in any of the modes described herein. Free-run EMG monitoring continuously listens for spontaneous muscle activity that might be indicative of potential danger. The system 10 may automatically cycle into free-run monitoring after 5 seconds of inactivity. Initiating a stimulation signal in the selected mode will interrupt the free-run monitoring until the system 10 has again been inactive for five seconds, at which time the free-run begins again.

The system 10 may test the integrity of pedicle holes (during and/or after formation) and/or screws (during and/or after introduction) via the Basic Stimulation EMG and Dynamic Stimulation EMG tests. To perform the Basic Stimulation EMG a test probe 116 is placed in the screw hole prior to screw insertion or placed on the installed screw head and a stimulation signal is applied. The insulating character of bone will prevent the stimulation current, up to a certain amplitude, from communicating with the nerve, thus resulting in a relatively high Iₜₕᵣₑₛₕ, as determined via the basic threshold hunting algorithm described below. However, in the event the pedicle wall has been breached by the screw or tap, the current density in the breach area will increase to the point that the stimulation current will pass through to the adjacent nerve roots and they will depolarize at a lower stimulation current, thus Iₜₕᵣₑₛₕ will be relatively low. The system described herein may exploit this knowledge to inform the practitioner of the current Iₜₕᵣₑₛₕ of the tested screw to determine if the pilot hole or screw has breached the pedicle wall.

In Dynamic Stim EMG mode, test probe 116 may be replaced with a clip 18 which may be utilized to couple a surgical tool, such as for example, a tap member 28 or a pedicle access needle 26, to the system 10. In this manner, a stimulation signal may be passed through the surgical tool and pedicle integrity testing can be performed while the tool is in use. Thus, testing may be performed during pilot hole formation by coupling the access needle 26 to the system 10, and during pilot hole preparation by coupling the tap 28 to the system 10. Likewise, by coupling a pedicle screw to the system 10 (such as via pedicle screw instrumentation), integrity testing may be performed during screw introduction.

In both Basic Stimulation EMG mode and Dynamic Stimulation EMG mode, the signal characteristics used for testing in the lumbar testing may not be effective when monitoring in the thoracic and/or cervical levels because of the proximity of the spinal cord to thoracic and cervical pedicles. Whereas a breach formed in a pedicle of the lumbar spine results in stimulation being applied to a nerve root, a breach in a thoracic or cervical pedicle may result in stimulation of the spinal cord instead, but the spinal cord may not respond to a stimulation signal the same way the nerve root would. To account for this, the surgical system 10 is equipped to deliver stimulation signals having different characteristics based on the region selected. By way of example only, when the lumbar region is selected, stimulation signals for the stimulated EMG modes comprise single pulse signals. On the other hand, when the thoracic and cervical regions are selected the stimulation signals may be configured as multipulse signals.

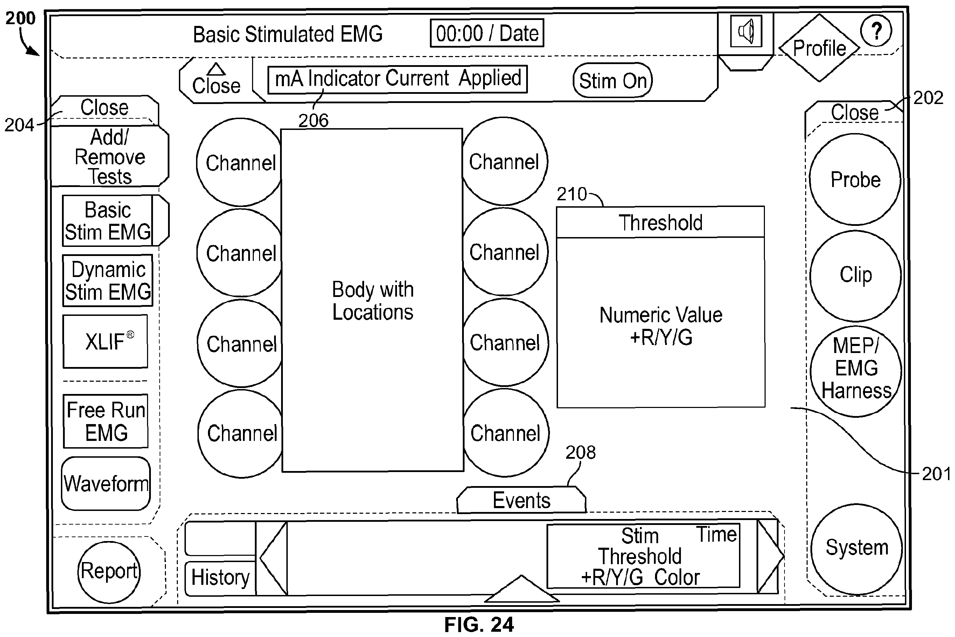

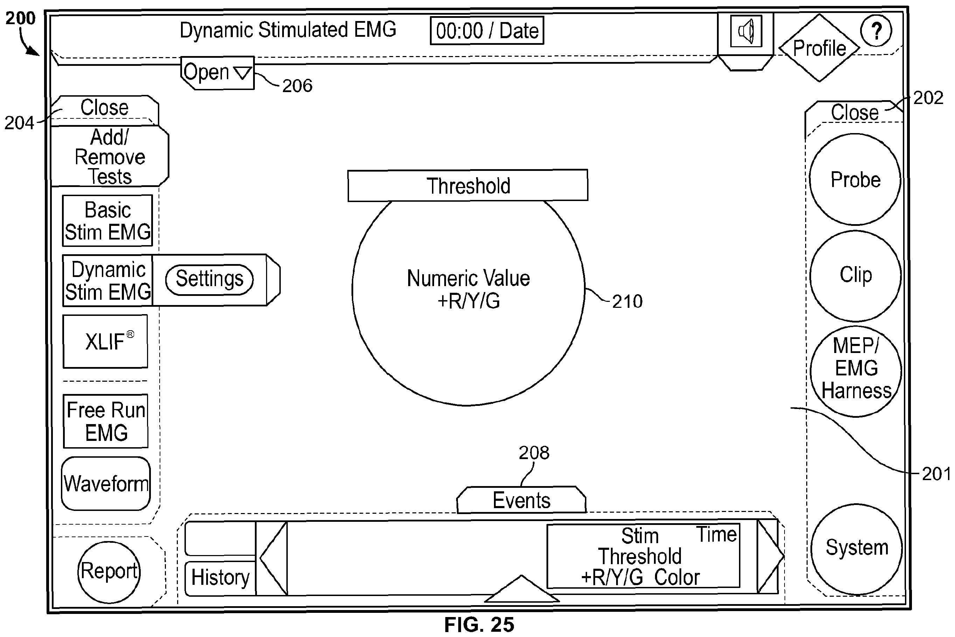

Stimulation results (including but not necessarily limited to at least one of the numerical Iₜₕᵣₑₛₕ value and color coded safety level indication) and other relevant data are conveyed to the user on at least main display 34, as illustrated in Figs. 24 and 25. Fig. 24 illustrates the monitoring screen 200 with the Basic Stimulation EMG test selected. Fig. 25 illustrates the monitoring screen 200 with the Dynamic Stimulation EMG test selected. In one example of the various screw test functions (e.g. Basic and Dynamic), green corresponds to a threshold range of greater than 10 milliamps (mA), a yellow corresponds to a stimulation threshold range of 7-10 mA, and a red corresponds to a stimulation threshold range of 6 mA or below. EMG channel tabs may be selected via the touch screen display 26 to show the Fᵣₑₛh of the corresponding nerves. Additionally, the EMG channel possessing the lowest 'ₜhᵣₑₛh may be automatically highlighted and/or colored to clearly indicate this fact to the user.

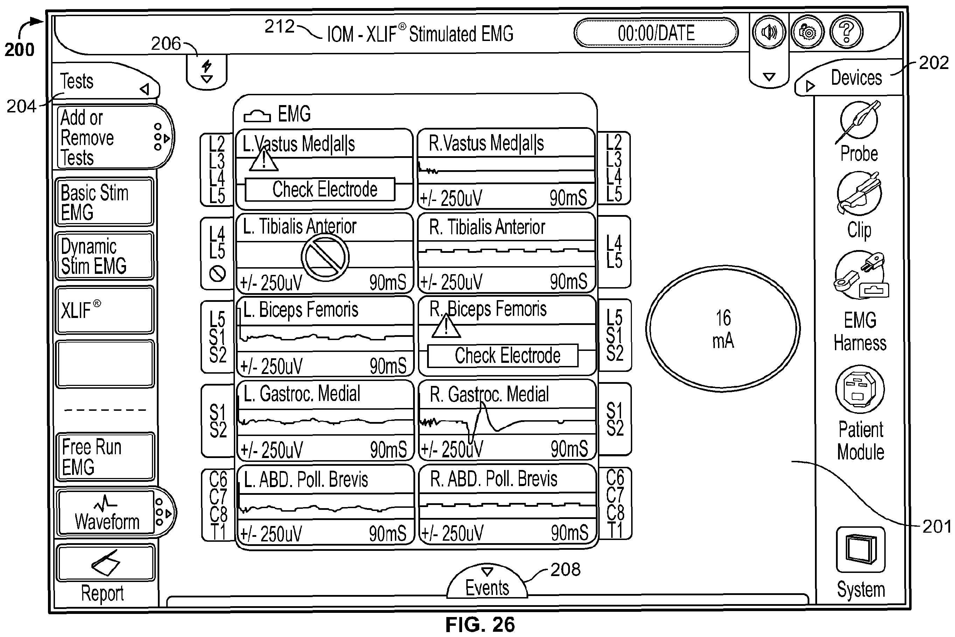

The system 10 may perform nerve proximity testing, via the XLIF mode, to ensure safe and reproducible access to surgical target sites. Using the surgical access components 26-32, the system 10 detects the existence of neural structures before, during, and after the establishment of an operative corridor through (or near) any of a variety of tissues having such neural structures which, if contacted or impinged, may otherwise result in neural impairment for the patient. The surgical access components 26-32 are designed to bluntly dissect the tissue between the patient's skin and the surgical target site. Dilators of increasing diameter, which are equipped with one or more stimulating electrodes, are advanced towards the target site until a sufficient operating corridor is established to advance retractor 32 to the target site. As the dilators are advanced to the target site electrical stimulation signals are emitted via the stimulation electrodes. The stimulation signal will stimulate nerves in close proximity to the stimulation electrode and the corresponding EMG response is monitored. As a nerve gets closer to the stimulation electrode, the stimulation current required to evoke a muscle response decreases because the resistance caused by human tissue will decrease, and it will take less current to cause nervous tissue to depolarize. 'ₜhᵣₑₛh is calculated, using the basic threshold hunting algorithm described below, providing a measure of the communication between the stimulation signal and the nerve and thus giving a relative indication of the proximity between access components and nerves. An example of the monitoring screen 200 with XLIF mode active is depicted in Fig. 26. In one example, a green or safe level corresponds to a stimulation threshold range of 10 milliamps (mA) or greater, a yellow level denotes a stimulation threshold range of 5-9 mA, and a red level denotes a stimulation threshold range of 4 mA or below.

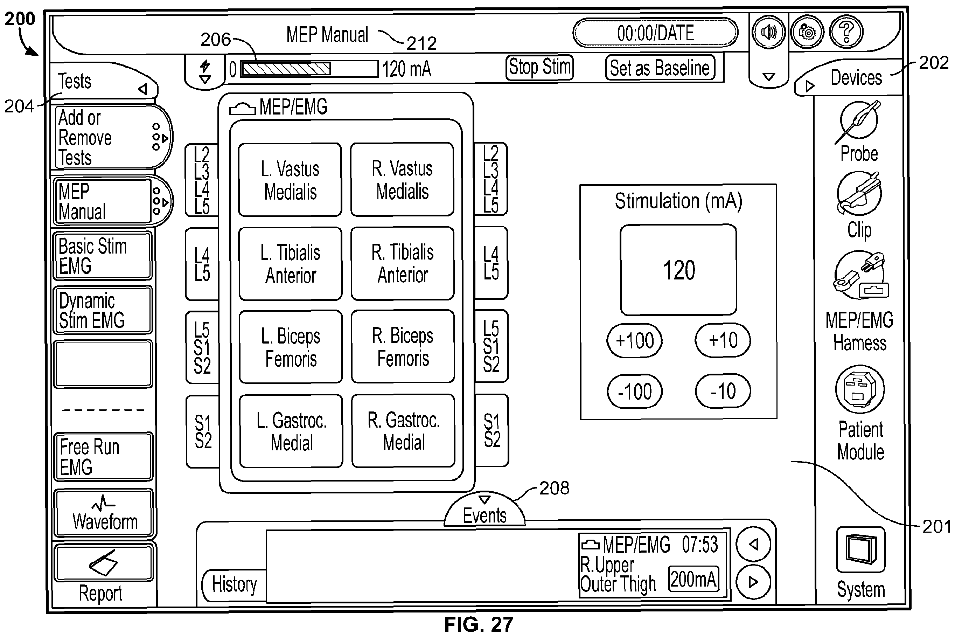

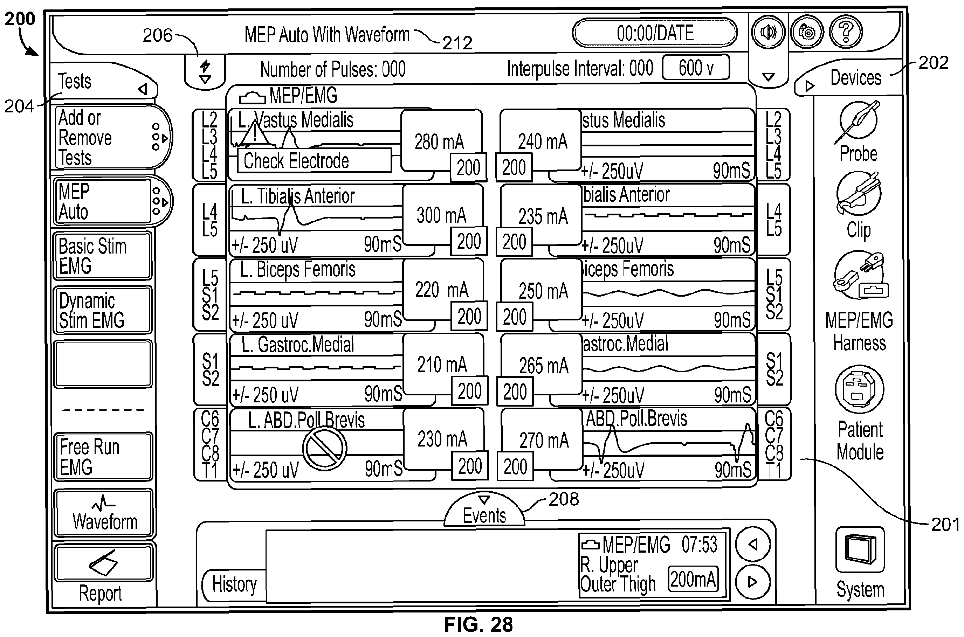

In MEP modes, stimulation signals are delivered to the motor cortex via patient module 14 and resulting responses are detected from various muscles in the upper and lower extremities. An increase in Iₜₕᵣₑₛₕ from an earlier test to a later test may indicate a degradation of spinal cord function. Likewise, the absence of a significant EMG response to a given Iₛₜᵢₘ on a channel that had previously reported a significant response to the same or lesser Iₛₜᵢₘ is also indicative of a degradation in spinal cord function. These indicators are detected by the system in the MEP modes and reported to the surgeon. In MEP Manual mode, the user selects the stimulation current level and the system reports whether or not the stimulation signal evokes a significant response on each channel. Stimulation results may be shown on the display 34 in the form of "YES" and "NO" responses, or other equivalent indicia, as depicted in Fig. 27. In MEP Automatic mode the system determines the Iₜₕᵣₑₛₕ baseline for each channel corresponding to the various monitored muscles, preferably early in the procedure, using the multi-channel algorithm described. Throughout the procedure subsequent tests may be conducted to again determine Iₜₕᵣₑₛₕ for each channel. The difference between the resulting Iₜₕᵣₑₛₕ values and the corresponding baseline are computed by the system 10 and compared against predetermined "safe" and "unsafe" difference values. The Iₜₕᵣₑₛₕ, baseline and difference values are displayed to the user, along with any other indicia of the safety level determined (such as a red, yellow, green color code), on the display 34, as illustrated in Fig. 28. Using either mode the surgeon may thus be alerted to potential complications with the spinal cord and any corrective actions deemed necessary may be undertaken at the discretion of the surgeon.

In Transcutaneous Nerve Root Stimulation modes, the system 10 is capable of ascertaining the health and/or status of at-risk nerves along the motor neural pathway superior and inferior to the surgical site before, during, and/or after the creation of the operative corridor to the surgical target site. To accomplish this, stimulation electrodes 22 may be placed on the skin over the desired spinal nerve roots (such as by way of example only, the L1 and L2 nerve roots and/or the location of the conus medullaris of the patient) and recording electrodes 24 are positioned on the recording sites (such as, by way of example only, the recording sites set forth above in Table 5). The control unit 12 and patient module 14 cooperate to transmit electrical stimulation signals to a stimulating cathode placed posteriorly on the patient's back. These stimulation signals cause nerves deep to the stimulating electrode to depolarize, evoking activity from muscles innervated by the nerves below. The system 10 detects and records the neuromuscular responses and optionally analyzes their relationship to the stimulation signal (discussed below). Resulting recording and/or assessment data is conveyed to the user on screen 200 and/or activator 330 as discussed herein. The TCNR testing described herein provides the ability to verify that the patient is positioned in a neutral way and that no neural structures have been impinged upon after the operative corridor has been established. In spinal surgery, for example, this is particularly advantageous in that the system 10 may be particularly suited for establishing an operative corridor to an intervertebral target site in a posterolateral, trans-psoas fashion so as to avoid the bony posterior elements of the spinal column.

According to the invention, a stimulating cathode is placed posteriorly and an anode is placed anteriorly at locations superior to the surgical target site and neuromuscular responses (transcutaneous nerve root "TCNR" responses) are evoked in response to transcutaneous, trans-abdominal motor pathway stimulation. By way of example only, the stimulating cathode may be a single cathode adhesive surface electrode placed over the conus medullaris at spinal level L1-2, preferably with the electrode pair oriented side to side and symmetrically over the neural foramen. The anode electrode may be an adhesive surface electrode placed at the anterior abdominal midline below the umbilicus, preferably with the electrode pair oriented side to side, symmetrically across the midline. Implementing a stimulation montage in this way is beneficial for at least two reasons. First, stimulating trans-abdominally does not evoke muscle twitching of the head, upper extremities, or upper torso which leads to less patient movement than transcranial MEP testing. Second, using a surface electrode with a larger surface electrode anteriorly and a smaller surface electrode posteriorly may decrease the current density travelling trans-abdominally, reducing the depolarization of the abdominal muscles and thus, further decreasing the amount of patient movement. Third, stimulating nerve roots with the TCNR techniques described herein can activate the specific area of the motor neural pathway of interest and elicit significant neuromuscular responses with a fixed polarity, single pulse stimulation whereas MEP testing requires multi-pulse trains of stimuli (oftentimes dual polarity stimulation as well) to activate the motor cortex and the entire corticospinal pathway to elicit clinically significant neuromuscular responses. This leads to greater specificity, less patient movement, and less power delivered to the patient. Recording electrodes may be placed on or in muscles innervated by one or more nerves of the lumbar plexus. The electrode harness 80 may be designed such that the various electrodes may be positioned about the patient as described in Table 5.

The steps of performing transcutaneous, trans-abdominal stimulation and recording the resultant evoked potentials is preferably first performed prior to establishing the lateral access corridor and subsequently performed periodically during the surgical procedure. In this way, the system 10 is capable of detecting changes to the stimulation threshold intensities of these nerves over time which may be indicative of changes to the health/status of these nerves (e.g. by compression or patient positioning). The system 10 may perform TCNR in either of two modes: Alert mode and Threshold mode. In Alert mode, the system 10 evaluates the neuromuscular responses for the presence/absence of a response. In Threshold mode, the system 10 detects changes to the stimulus intensity required to elicit a significant response. By way of example only, a change in the health or status of a nerve may be deemed significant once the stimulus intensity required to elicit a neuromuscular response from a muscle exceeds pre-determined criteria (e.g. 200 A greater than the baseline stimulation threshold). The system 10 may quickly and accurately determine this data and convey the useful information in a simple and easily comprehensible manner for interpretation by a surgeon, neurophysiologist, or other medical personnel. It is contemplated that the control unit 12 of the system 10 may automatically ascertain this information and/or communicate any of numerical, graphic, audio, and visual feedback corresponding to one or more of these findings. Armed with this useful information, the surgeon may detect a problem or potential problem early and then act to avoid and/or mitigate the problem.

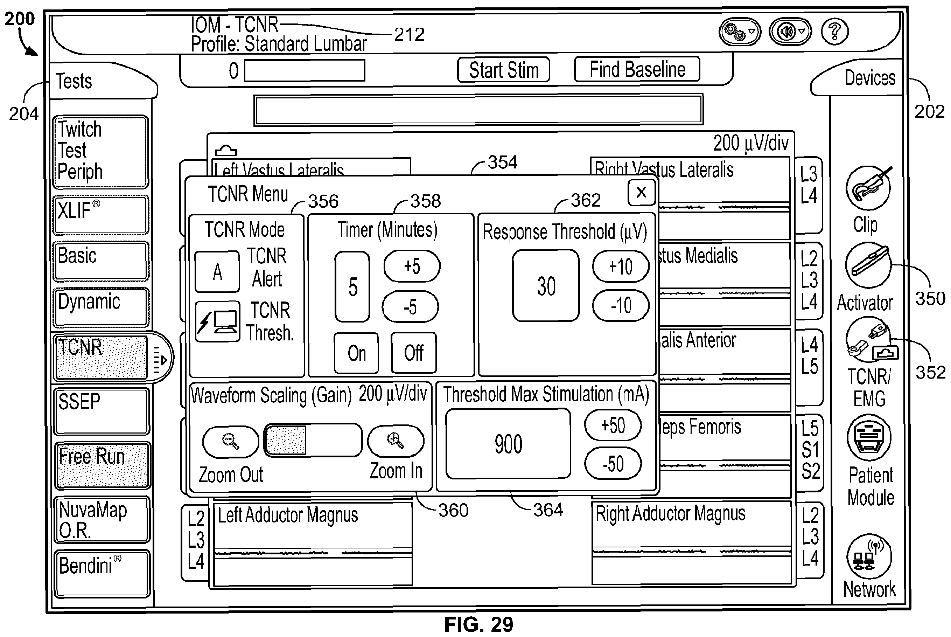

Figs. 29-34 are example screen displays of the TCNR mode according to one example of the system 10. Fig. 29 illustrates an intraoperative monitoring (IOM) setup screen from which various features and parameters of the TCNR mode may be controlled and/or adjusted by the user as desired. It is contemplated that there are any number of ways that the TCNR mode and TCNR testing may be activated by the user. For example, the user may select the TCNR mode from the test selection tab 204. The user may plug the activator 330 into accessory port 133 as described above. The user may press stimulation button 340 on activator 330 which will automatically toggle the system 10 from the mode it was performing prior to TCNR mode selection to TCNR mode (e.g. XLIF mode). According to some implementations, pressing stimulation button 340 in this manner 340 not only toggles the system 10 to TCNR mode, but also immediately initiates a TCNR stimulation test as well giving the user rapid flexibility in assessing the health and status of various aspects of the lower motor neural pathway. As shown in Fig. 29, the devices tab 202 indicates to the user an activator icon 350 and a TCNR/EMG icon 350 indicating that the activator 330 and TCNR/EMG harness 80 are properly connected to the system 10.

Selecting the TCNR button from the test selection tab 204 brings up a TCNR Menu window 354. The user may toggle between Alert mode and Threshold mode in TCNR Mode field 356, and change one or more profile settings (e.g. timer, waveform scaling, response threshold maximum stimulation). As will be explained in detail below, in some implementations, a user may wish to know when a predetermined period of time has elapsed between TCNR stimulations. According to one or more examples, there is provided a timer to address such a need. The timer setting may be adjusted in timer field 358. The timer may be turned on or off and the time may be selected from a range between 0 and 30 minutes, with a default value of 5 minutes. The timer setting may be increased or decreased in increments of 5 minutes using the timer selection buttons labeled (by way of example only) "+5" and "-5". Waveform scaling may be accomplished by increasing or decreasing the gain by using the buttons labeled (by way of example only) "Zoom In" and "Zoom Out" in waveform scaling field 360. According to one example, the gain has a range of 10µV and 10,000µV with a default gain setting is 200µV. Each adjustment increases or decreases the gain by a fixed increment (e.g. 10µV). The minimum threshold setting for which a given response qualifies as a response may be displayed and adjusted in response threshold field 362. The selected response threshold setting may be increased or decreased in increments of 10µV using the buttons labeled "+10" and "-10". According to one example, the threshold setting may be selected from a range of 10µV to 300µV with a default value of 30µV. The maximum stimulation intensity that may be delivered in TCNR Threshold mode may also be selected in Threshold maximum stimulation field 364. According to one example, the maximum threshold may be selected from a range between SOmA and 1500mA with a starting value of 100mA. The threshold setting may be increased or decreased in increments of 50A using buttons labeled (by way of example only) "+50" and "-50". As shown by way of example in Fig. 29, the maximum threshold is shown at 900mA, that is to say the maximum threshold that may be delivered to the patient is 900mA.

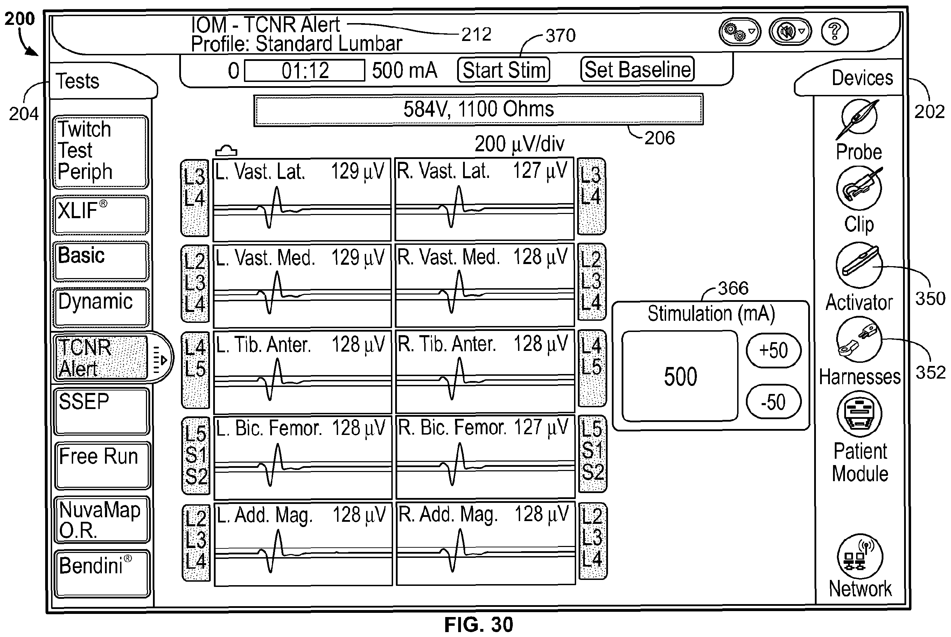

Figs. 30-31 depict example screen displays for the Alert mode of the TCNR monitoring function. These screen displays are intended to communicate information to the surgeon or other personnel in an easy-to-interpret fashion. This information may include, but is not necessarily limited to, a display of the test mode 212, channels 10 for each myotome [indicating]: spinal levels monitored (e.g. L. Add. Mag.); waveforms of the evoked responses, voltages of the evoked responses; a stimulation bar 206 to display particulars of the stimulation (e.g. stimulation parameters, the stimulation intensity required to elicit a response, and the stimulation intensity required to elicit a response. This information may be communicated in any number of suitable fashions, including but not limited to, the use of visual indicia (such as alpha-numeric characters, light-emitting elements, and/or graphics) and audio communication (such as a speaker element).

In TCNR Alert mode, the underlying neurophysiologic principle of operation is to assess the health and status of the lower motor neural pathway via the presence/absence of evoked neuromuscular responses for each muscle monitored. To perform TCNR monitoring in Alert mode, the user first manually selects the stimulation intensity to be delivered to the stimulation electrodes. The stimulation intensity that may be delivered in TCNR Alert mode may also be selected in Alert stimulation field 366. According to one example, the simulation intensity may be selected from a range between 50mA and 1500mA with a starting value of 100mA. The setting may be increased or decreased in increments of 50mA with a starting value of 100mA. The setting may be increased or decreased in increments of 50mA using buttons labeled (by way of example only) "+50" and "-50". As shown by way of example in Fig. 30, the stimulation intensity to be delivered is 500mA.

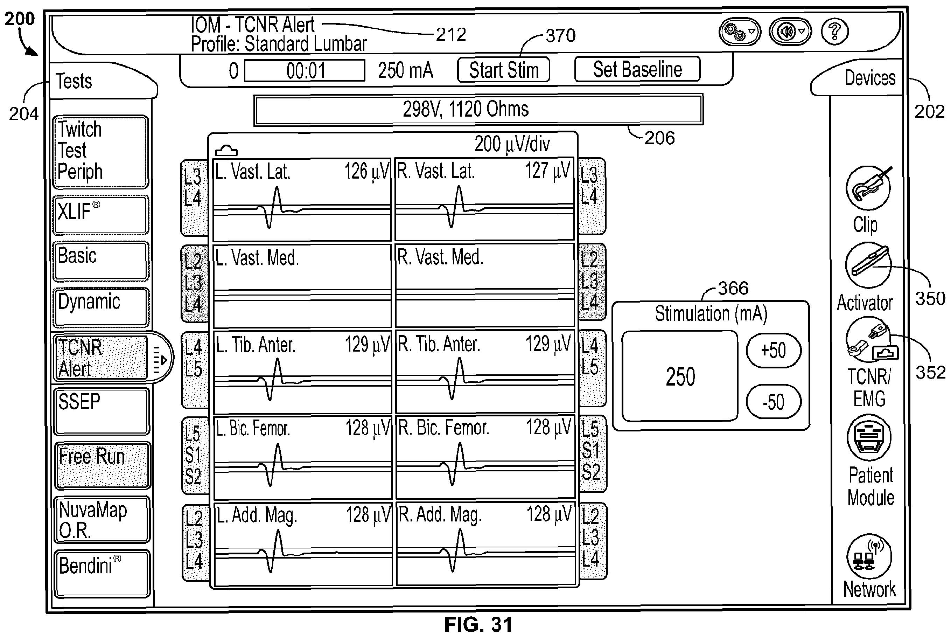

According to one or more implementations, for a given stimulation intensity (mA) the presence of a significant EMG response for a particular muscle is presented to the user via a green color indicator and the absence of a significant EMG response for a particular muscle is presented to the user via a red color indicator. As shown in Fig. 30, responses were obtained in all 10 channels for all muscles with 500mA stimulation. As such, a green color indicator is shown in each channel 110. Referring now to Fig. 31, responses were obtained in all channels except the left and right vastus medialis channels with 250mA stimulation. As such, red color indicators are shown in the left and right vastus medialis channels and green color indicators are shown in the remaining channels in which a significant response was recorded. According to some examples, the system 10 provides visual and audible alerts when the responses are absent. By way of example, the system 10 may flash a warning on screen 200, or an audible tone.

In TCNR Alert mode, periodic testing for the presence/absence of TCNR responses are made to monitor the health and status of the lower motor neural pathways. As indicated above, a user may which to know when a predetermined period of time has elapsed between TCNR tests. Armed with such information, the user may perform another TCNR test or wait until a later time in the surgical procedure to do so. The timer interval may be set as described above. After each TCNR test, the system 10 will initiate a timer corresponding to the selected time interval and, when the time has elapsed, the system 10 will activate a reminder alert. The reminder alert may include, by way of example only, any one of, or combination of, an audible tone, voice recording, screen flash, pop up window, scrolling message, or any other such alert to remind the user to perform a TCNR test again. The alerts may be displayed on screen 200 (for example, in the stimulation bar), on the activator (for example via LED indicator 342), or both screen 200 and activator 330 simultaneously.

For optimal consistency of TCNR testing and the information it provides, the TCNR stimulations are preferably conducted at a set time interval throughout the surgical procedure (e.g., every 5 minutes). According to one example, after 5 minutes have elapsed, the LED indicator 342 and/or the on-screen stimulator bar 206 blinks green at a 1Hz rate, The user may decide to perform another TCNR test either by pressing button 340 or "Start Stim" button 370 or the user may decide to wait until a later time in the surgical procedure. After 10 minutes have elapsed, LED indicator 342 (and/or the on-screen stimulator bar 206) continues to blink green but at a 2Hz rate. When the user decides to stimulate, he/she presses the activator stimulator button 340 or the "Start Stim" button 370 to run the TCNR test and the timer function restarts. While described herein with respect to the TCNR Alert mode, it is contemplated that the timer function may be implemented in TCNR Threshold mode as well.

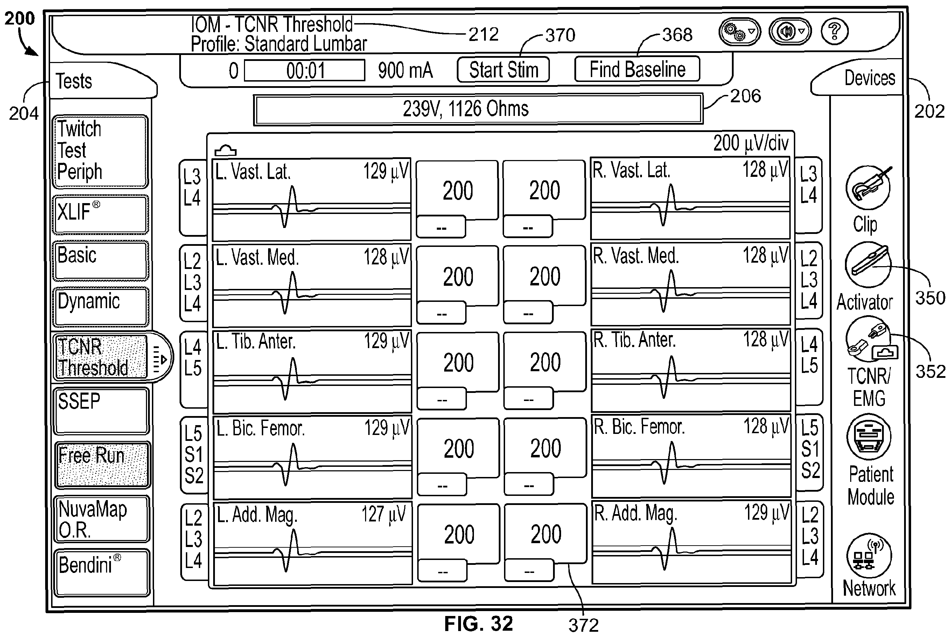

Figs. 32-34 depict example screen displays for the Threshold mode of the TCNR monitoring function. In TCNR Threshold mode, the underlying neurophysiologic principle is that, in order to monitor the health of the lumbar motor neural pathway, the user must be able to determine if the evoked responses are changing with respect to the stimulation signal. To monitor for this change, baseline TCNR responses are established for all muscles of interest (preferably prior to surgical manipulation) and then compared to subsequent TCNR responses periodically throughout the procedure.

The baseline feature of the TCNR Threshold mode will now be described with respect to Fig. 32. Selecting the "Find Baseline" button 368 on the screen 200 or pressing the stimulation button 340 on the activator 330 starts the baseline stimulation sequence. The system 10 then delivers one or more stimulation pulses and/or trains of pulses until a baseline TCNR response is evoked in one or all muscle channels. By way of example only, the system 10 may be configured to determine the stimulus response threshold (Iₜhᵣₑₛh) via one or more of the threshold hunting and thresholding algorithms described below. Fig. 32 depicts an example set of responses in which baseline TCNR responses and baseline response thresholds (Inuesh-Base) were found in all 10 muscle channels (shown for each channel as 200mA in results field 372). According to some examples, baseline TCNR response waveforms remain in the waveform windows for comparison to subsequent stimulation trials. This allows for more detailed comparison of the response waveform amplitudes and morphologies. The peak to peak amplitude measurements (in microvolts) of both the baseline and subsequent results are displayed in the waveform window. In some implementations, the baseline waveforms and subsequent waveforms bay be indicated differently, by way of example the baseline waveform may be depicted as purple and the current waveform may be depicted as white.

In the event the system detects a significant increase in the amount of stimulus intensity required to elicit a neuromuscular response (Imeth), or if no neuromuscular response is obtained at the maximum stimulus intensity permitted, the associated window may preferably be highlighted with a predetermined color (e.g. red) to indicate the potential danger to the surgeon. Preferably, the stimulation results are displayed to the surgeon along with a color code so that he/she may easily comprehend the danger, diagnose, and take corrective measures to avoid or mitigate such danger. This may, for example, more readily permit TCNR monitoring results to be interpreted by the surgeon or assistant without necessarily requiring dedicated monitoring personnel. By way of example only, red is used when the increase in threshold stimulus intensity is within a predetermined safe level. Yellow is used when the increase in threshold stimulus intensity is between the predetermined safe and unsafe levels. By way of example only, the system 10 may also notify the user of potential danger through the use of a warning which may be communicated to the user by any one of, or combination of, a pop-up window, an audible tone, voice recording, screen flash, scrolling message, or any other such alert to notify the user of potential danger. The system 10 may notify the user according to the following scenario:

**Table 9:**

| | | | |
|---|---|---|---|
| Change in _{'Thresh} from Baseline | Color | Audio Feedback | Neurophysiologic Assessment |
| 0-100mA | Green | Slow, Low Pitch | No significant change from baseline |
| > 100mA to < 200mA | Yellow | Faster, Higher Pitch | Noteworthy change from baseline |
| 200mA or more | Red | Fastest, Highest Pitch | Significant change from baseline |

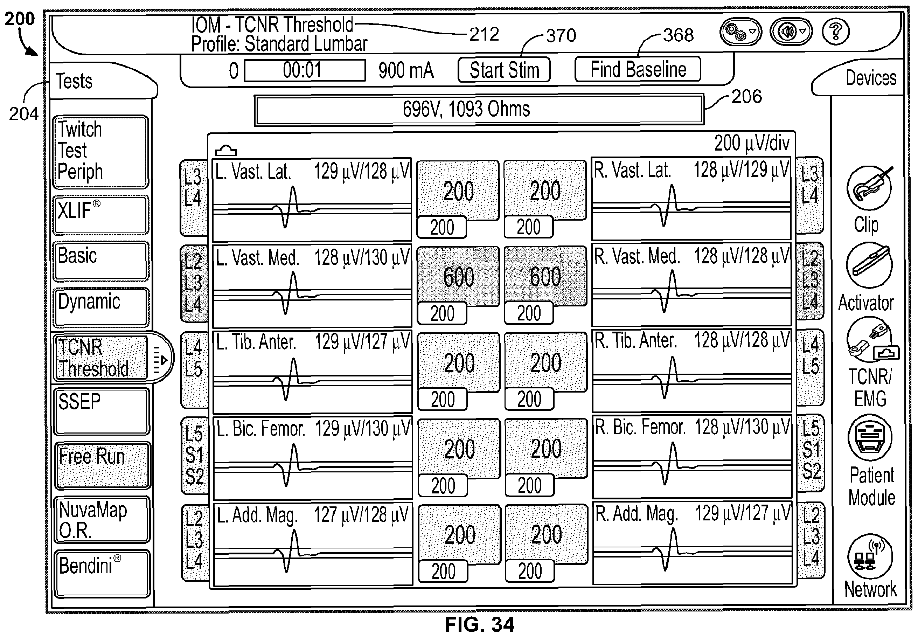

Figs. 33-34 depict example TCNR responses obtained subsequent to the baseline TCNR responses of Fig. 32. The baseline Iₜₕᵣₑₛₕ response results move from results field 372 to baseline field 374. As shown in Fig. 33, responses in a subsequent test were obtained in all channels 110 with 200mA stimulation (I_{thresh-subsequent}), indicating no significant changes in neurophysiologic recordings from baseline. These results are displayed in results field 372. As such, green color indicators shown for each of these channels in accordance with the example shown and
described above in Table 9. Referring now to Fig. 34, responses in another subsequent trial were obtained in all channels, 200mA of stimulation intensity was required to elicit a significant neuromuscular response in the bilateral vastus lateralis, tibialis anterior, biceps femoris, and adductor magnus channels indicating no significant changes in neurophysiologic recordings from baseline. Green color indicators are shown for each channel. However, 600mA of stimulation intensity was required to elicit a significant neuromuscular response in the bilateral vastus medialis channels which is a 400mA increase from the baseline requirement of 200mA. Red color indicators are shown for each of these channels. It is to be appreciated that the predetermined safety levels, colors, audio feedback, and neurophysiologic interpretation set forth above are merely for illustrative purposes only and are not meant to be limiting in any way.

In some surgical procedures, it may be advantageous to perform more than one type of neurophysiologic testing during a surgical procedure. Oftentimes, it is advantageous to perform multiple types of neurophysiologic testing and monitoring modes intermittently during a procedure. Constantly switching between modes and initiating/re-initiating testing in each mode can be cumbersome, time-consuming, and frustrating for users. They may become impatient with the system 10. In accordance with the present invention, the system 10 includes functionality to toggle or switch between two or more neurophysiologic monitoring modes quickly and seamlessly as will be discussed below.

For illustrative purposes only, in a minimally-invasive lateral approach spine procedure (e.g., XLIF^{®}), it may be desirable to perform both nerve proximity testing and transcutaneous nerve root testing intermittently, but at different times from one another throughout the surgical procedure. The control unit 12 possesses the requisite functionality to toggle or switch between XLIF and TCNR modes as directed by the user. In some examples, the toggling or switching is effectuated by the stimulation clip 18/activator 330 assembly described above. According to some implementations, if the user has selected XLIF mode, the system 10 will stay in XLIF mode until an indication from the user. This indication may be pressing the stimulation button 340 on the activator 330. Pressing the stimulation button 340 one time will simultaneously toggle to the appropriate TCNR mode screen and perform a stimulation trial in TCNR mode. To toggle or switch back to XLIF mode, the user may press activation button 131 on button module 129 to simultaneously toggle to the XLIF mode screen and perform a stimulation trial in XLIF mode or, if a stimulation trial in XLIF mode is not desired at that time, the user can do nothing. The TCNR screen may time out (for example, 1 minute after the last TCNR stimulation trial) and the system 10 will toggle or switch back to the XLIF mode screen 200. However, the TCNR timer function will still be active and will remind the user to perform another stimulation when the timer has lapsed as set forth above. Thus, the user is able to perform more than one neurophysiologic monitoring modes intermittently without having to does not need to make/or instruct hospital personnel to make changes on the screen 200 to switch between modes.

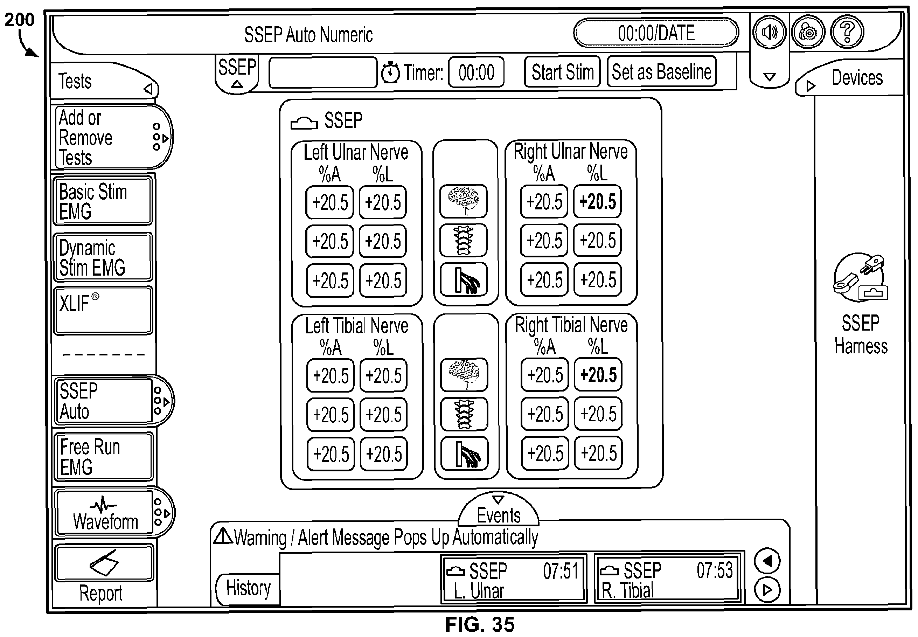

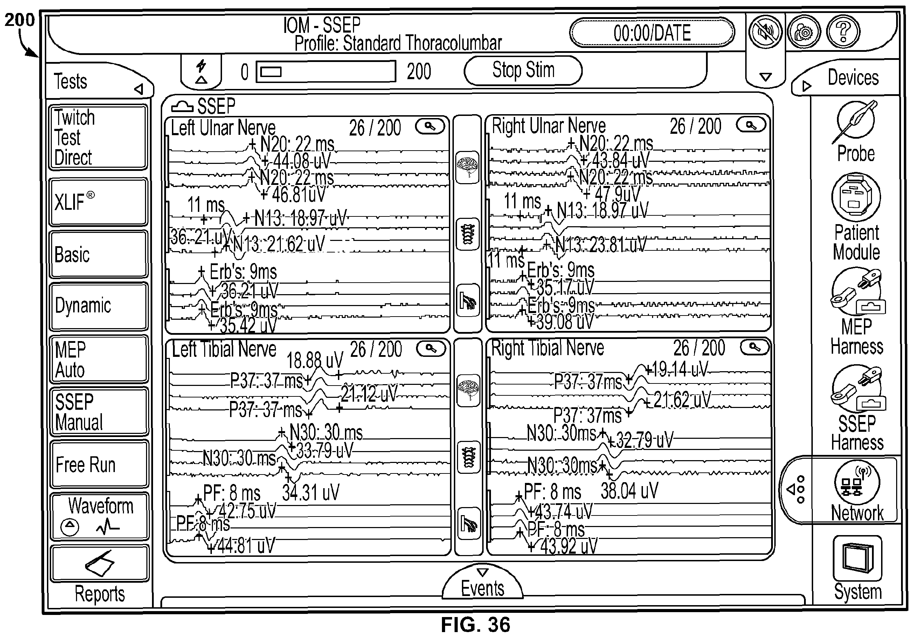

In the SSEP Manual and Automatic modes, stimulation signals are delivered to peripheral sensory electrodes placed over the desired peripheral nerve (such as, by way of example only, the Posterior Tibial nerve and/or the Ulnar nerve) and recording electrodes 23 are positioned on or over the recording sites (such as, by way of example only, over the C2 vertebra, scalp, Erb's Point, and Popliteal Fossa), and stimulation signals are delivered from the patient module14. Damage in the spinal cord may disrupt the transmission of the signal up the spinal cord resulting in a weakened or delayed signal at the recording site. In SSEP Manual mode, the signal response waveforms and latency values associated with those waveforms are displayed for the user. The user then makes a comparison between a baseline signal response and a signal response, as depicted in Fig. 35. In SSEP Automatic mode, the system 10 compares the difference between the amplitude and latency of the signal response vs. the amplitude and latency of a baseline signal response. These differences are compared against the predetermined "safe" and "unsafe" levels and the results are displayed on display 34. The results are displayed to the user along with any other indicia of the safety level determined (such as a red, yellow, green color code), on the display 34, as illustrated in Fig. 36). The system 10 may employ one or more algorithms to quickly select the optimal stimulus parameters for conducting SSEP testing on each active stimulation channel. This can be done according to any number of algorithms that automatically adjust various parameters until a combination resulting in the most desirable result is achieved.

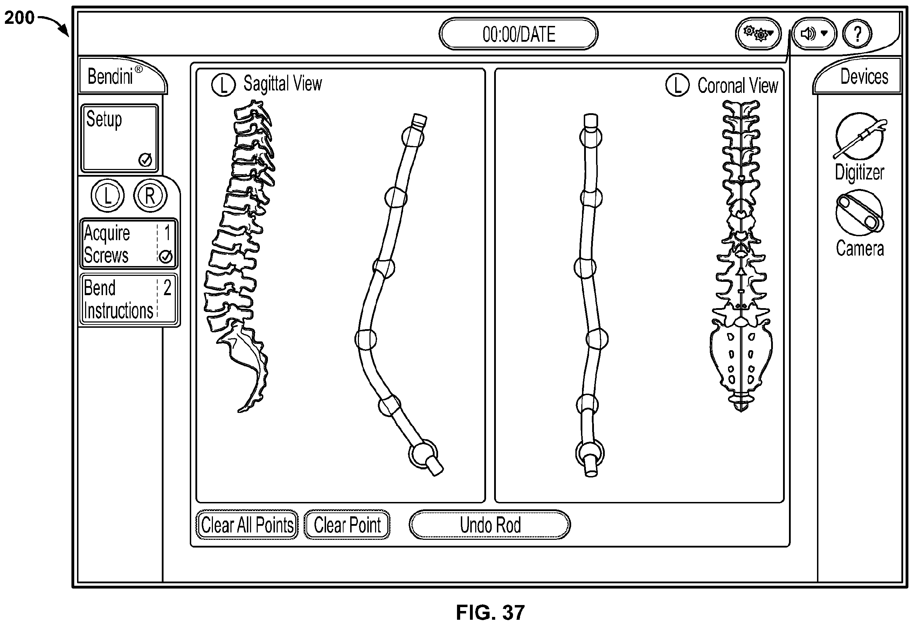

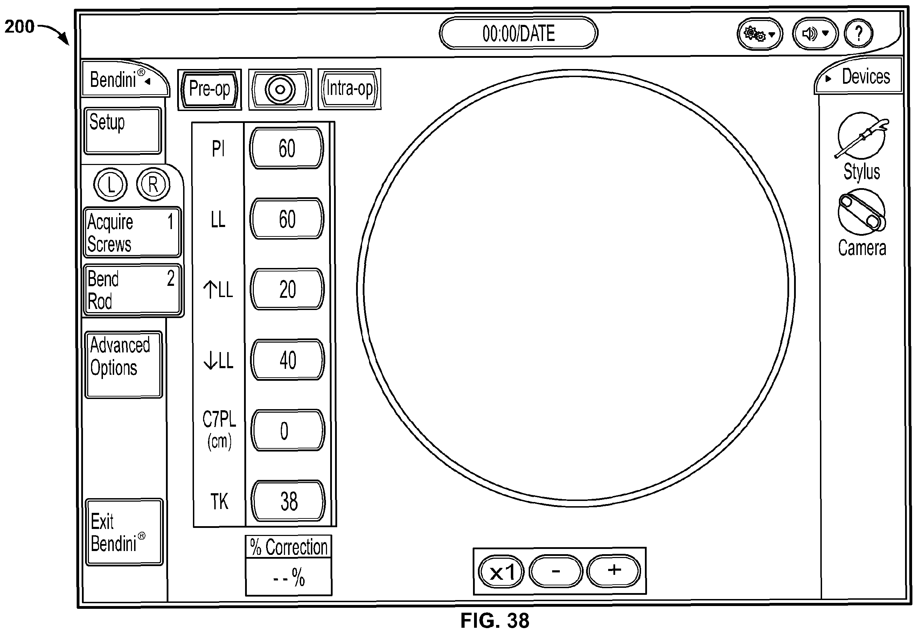

In the surgical correction planning and assessment mode, the system 10 aids the user in planning and assessing the degree to which he/she has achieved the surgical goals during a spinal procedure. As illustrated, by way of example only, in Fig. 37, there may be included a feature in which the system 10 is capable of digitizing implanted screw positions, outputting bend instructions for a rod, and previewing the shape of the rod on screen 200. In some implementations, the rod bending instructions are for a rod shaped to custom-fit within the implanted screw locations. In other implementations, the system 10 is further capable of accepting correction inputs via one or more advanced option features for viewing bend instructions for a rod shaped to fit at locations apart from those implanted screw locations. Installing a rod shaped in this manner could correct a curvature deformity in the patient's spine according to a user's prescribed surgical plan. In some spinal procedures, restoring a patient's spine to a balanced position may be a desired surgical outcome. As shown in Fig. 38, there may also be included a feature in which the system 10 is configured to receive and assess 1) preoperative spinal parameter inputs; 2) target spinal parameter inputs; 3) intraoperative spinal parameter inputs; and/or postoperative spine parameter inputs via screen 200. Spinal parameters may comprise the patient's Pelvic Incidence (PI), Pelvic Tilt (PT), Sacral Slope (SS), Lumbar Lordosis (LL), Superior Lumbar Lordosis (I'LL), Inferior Lumbar Lordosis (ILL), C7 Plumb Line Offset (C7PL), Thoracic Kyphosis (TK), T1 Tilt, and Sagittal, Vertical Axis (SVA) measurements. One or more of these inputs may be tracked and/or compared against other inputs to assess how the surgical correction is progressing toward a surgical plan and utilized to develop/refine an operative plan to achieve the desired surgical correction.

To obtain 'thresh and take advantage of the useful information it provides, the system 10 identifies and measures the peak-to-peak voltage (Vₚₚ) of each EMG response corresponding to a given stimulation current (Iₛₜᵢₘ.). Identifying the true vpp of a response may be complicated by the existence of stimulation and/or noise artifacts which may create an erroneous vpp measurement. To overcome this challenge, the system 10 of the present invention may employ any number of suitable artifact rejection techniques such as those shown and described in full in the above referenced co-pending and commonly assigned PCT App. Ser. No. PCT/US2004/025550, entitled "System and Methods for Performing Dynamic Pedicle Integrity Assessments," filed on August 5, 2004.

Upon measuring vpp for each EMG response, the Vₚₚ information is analyzed relative to the corresponding stimulation current (Iₛₜᵢₘ) in order to identify the minimum stimulation current (I_{Thresh}) capable of resulting in a predetermined vpp EMG response. According to the present invention, the determination of I_{Thresh} may be accomplished via any of a variety of suitable algorithms or techniques.

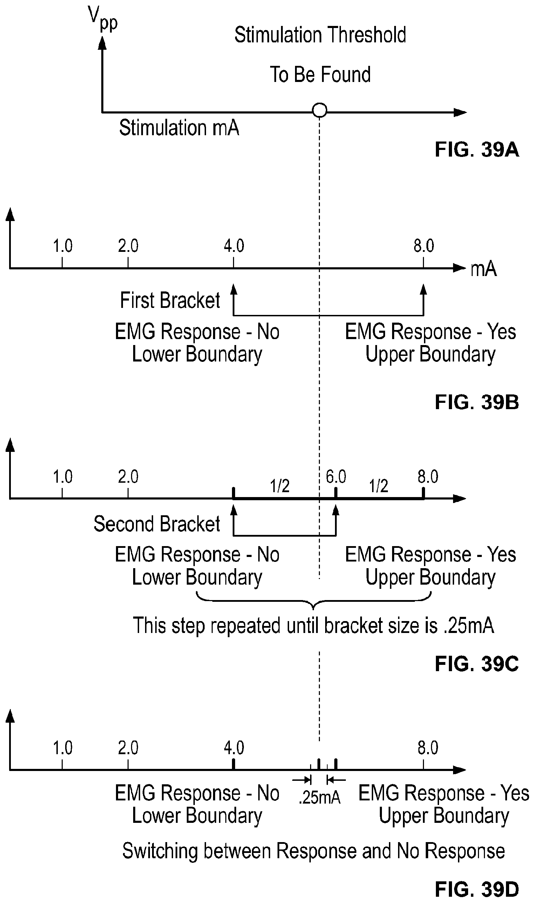

Figs. 39 A-D illustrate, by way of example only, the principles of a threshold hunting algorithm of the present invention used to quickly find Ithresh. The method for finding Iₜₕᵣₑₛₕ utilizes a bracketing method and a bisection method. The bracketing method quickly finds a range (bracket) of stimulation currents that must contain I_{Thresh} and the bisection method narrows the bracket until I I_{Thresh} is known within a specified accuracy. If the stimulation current threshold, I_{Thresh}, of a channel exceeds a maximum stimulation current, that threshold is considered out of range.

Figs. 39 A-D illustrate the bracketing feature of the threshold hunting algorithm of the present invention. Stimulation begins at a minimum stimulation current, such as (by way of example only) lmA. It will be appreciated that the relevant current values depend in part on the function performed (e.g. high currents are used for MEP and low currents are generally used for other functions) and the current values described here are for purposes of example only and may in actuality be adjusted to any scale. The level of each subsequent stimulation is doubled from the preceding stimulation level until a stimulation current recruits (i.e. results in an EMG response with a vpp greater or equal to V ₜₕᵣₑₛₕ). The first stimulation current to recruit (8 mA in Fig. 39 B), together with the last stimulation current to have not recruited (4 mA in Fig. 39 B), forms the initial bracket.

Figs. 39 C-D illustrate the bisection feature of the threshold hunting algorithm of the present invention. After the threshold current Iₜhᵣₑₛh has been bracketed (Fig. 39 B), the initial bracket is successively reduced via bisection to a predetermined width, such as (by way of example only) 0.25 mA. This is accomplished by applying a first bisection stimulation current that bisects (i.e. forms the midpoint of) the initial bracket (6 mA in Fig. 39 C). If this first bisection stimulation current recruits, the bracket is reduced to the lower half of the initial bracket (e.g. 4 mA and 6 mA in Fig. 39 C). If this first bisection stimulation current does not recruit, the bracket is reduced to the upper half of the initial bracket (e.g. 6 mA and 8 mA in Fig, 39 C). This process is continued for each successive bracket until Iₜₕᵣₑₛₕ is bracketed by stimulation currents separated by the predetermined width (which, in this case, is 0.25 mA). In this example shown, this would be accomplished by applying a second bisection stimulation current (forming the midpoint of the second bracket, or 5 mA in this example). Because this second bisection stimulation current is below Mesh, it will not recruit. As such, the second bracket will be reduced to the upper half thereof (5 mA to 6 mA), forming a third bracket. A third bisection stimulation current forming the mid-point of the third bracket (5.50 mA in this case) will then be applied. Because this third bisection stimulation current is below Iₜₕᵣₑₛₕ, it will not recruit. As such, the third bracket will be reduced to the upper half thereof (5.50 mA to 6 mA), forming a fourth bracket. A fourth bisection stimulation current forming the mid-point of the fourth bracket (5.75 mA in this case) will then be applied. Because the fourth bisection stimulation current is above Iₜₕᵣₑₛₕ, it will recruit. The final bracket is therefore between 5.50mA and 5.75 mA. Due to the "response" or recruitment at 5.50 mA and "no response" or lack of recruitment at 5.75 mA, it can be inferred that Iₜₕᵣₑₛₕ is within this range. In one example, the midpoint of this final bracket may be defined as Iₜₕᵣₑₛₕ, however, any value falling within the final bracket may be selected as Iₜₕᵣₑₛₕ without departing from the scope of the present invention. Depending on the active mode, the algorithm may stop after finding Mesh for the first responding channel (i.e. the channel with the lowest I thresh) or the bracketing and bisection steps may be repeated for each channel to determine Iₜₕᵣₑₛₕ for each channel. In one example, this multiple channel Iₜₕᵣₑₛₕ determination may be accomplished by employing the additional steps of the multi-channel threshold detection algorithm, described below.

Additionally, in the "dynamic" functional modes, including, but not necessarily limited to Dynamic Stimulation EMG and XLIF, the system may continuously update the stimulation threshold level and indicate that level to the user. To do so, the threshold hunting algorithm does not repeatedly determine the Iₜₕᵣₑₛₕ level anew, but rather, it determines whether stimulation current thresholds are changing. This is accomplished, as illustrated in Fig. 39 D, by a monitoring phase that involves switching between stimulations at lower and upper ends of the final bracket. If the threshold has not changed then the lower stimulation current should not evoke a response, while the upper end of the bracket should. If either of these conditions fail, the bracket is adjusted accordingly. The process is repeated for each of the active channels to continue to assure that each threshold is bracketed. If stimulations fail to evoke the expected response three times in a row, then the algorithm transitions back to the bracketing state in order to reestablish the bracket. In the event a change in Mesh is detected during the monitoring phase, the user may be alerted immediately via the screen display and/or audio feedback. By way of example only, the color shown on the display corresponding to the previous 'ₜhᵣₑₛh can be altered to a neutral color (e.g. black, grey, etc...) as soon as the change in ' Iₜₕᵣₑₛₕ is detected but before the new 'thresh value is determined, If an audio tone is used to represent a particular safety level, the tone can ceased as soon as the change in detected. Once the new thresh value is determined the color and/or audio tone can be altered again to signify the value.

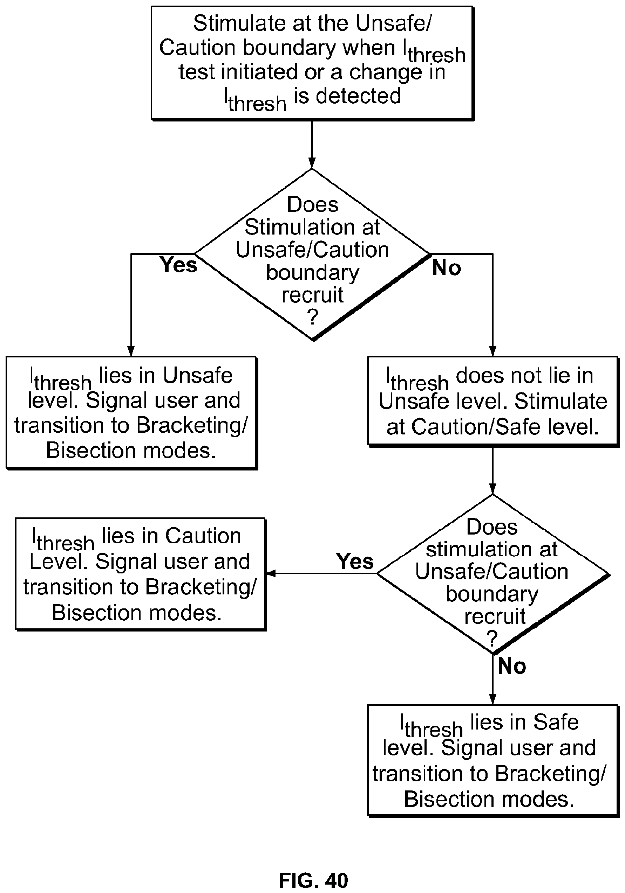

In an alternative, rather than beginning by entering the bracketing phase at the minimum stimulation current and bracketing upwards until 'ₜhᵣₑₛh is bracketed, the threshold hunting algorithm may begin by immediately determining the appropriate safety level and then entering the bracketing phase. The algorithm may accomplish this by initiating stimulation at one or more of the boundary current levels. By way of example only, and with reference to Fig. 40, the algorithm may begin by delivering a stimulation signal at the boundary between the unsafe (e.g. red) and caution (e.g. yellow) levels. If the safety level is not apparent after the first stimulation, the algorithm may stimulate again at the boundary between the caution (e.g. yellow) and safe (e.g. green) levels. Once the safety level is known (i.e. after the first stimulation if the safety level is red, or, after the second stimulation if the safety level is yellow or green) the screen display may be updated to the appropriate color and/or coded audio signals may be emitted. As the screen display is updated, the algorithm may transition to the bracketing and bisection phases to determine the actual 'thresh value. When the thresh value is determined the display may be updated again to reflect the additional information. In dynamic modes, if the monitoring phase detects a change in Iₜₕᵣₑₛₕ, the algorithm will again stimulate at the boundary level(s) as necessary and update the color and/or audio signals before transitioning to the bracketing and bisection phases to determine the new Iₜₕᵣₑₛₕ.

For some functions, such as (by way of example) MEP, it may be desirable to obtain _{I} Iₜₕᵣₑₛₕ for each active channel each time the function is performed. This is particularly advantageous when assessing changes in 'thresh over time as a means to detect potential problems (as opposed to detecting an Iₜₕᵣₑₛₕ below a predetermined level determined to be safe, such as in the Stimulated EMG modes). While Iₜₕᵣₑₛₕ can be found for each active channel using the algorithm as described above, it requires a potentially large number of stimulations, each of which is associated with a specific time delay, which can add significantly to the response time. Done repeatedly, it could also add significantly to the overall time required to complete the surgical procedure, which may present added risk to the patient and added costs. To overcome this drawback, an example of the system 10 boasts a multi-channel threshold hunting algorithm so as to quickly determine Iₜₕᵣₑₛₕ for each channel while minimizing the number of stimulations and thus reduce the time required to perform such determinations.

The multi-channel threshold hunting algorithm reduces the number stimulations required to complete the bracketing and bisection steps when Iₜₕᵣₑₛₕ is being found for multiple channels. The multi-channel algorithm does so by omitting stimulations for which the result is predictable from the data already acquired. When a stimulation signal is omitted, the algorithm proceeds as if the stimulation had taken place. However, instead of reporting an actual recruitment result, the reported result is inferred from previous data. This permits the algorithm to proceed to the next step immediately, without the time delay associated with a stimulation signal.

Regardless of what channel is being processed for thresh, each stimulation signal elicits a response from all active channels. That is to say, every channel either recruits or does not recruit in response to a stimulation signal (again, a channel is said to have recruited if a stimulation signal evokes an EMG response deemed to be significant on that channel, such as vpp of approximately 100 uV). These recruitment results are recorded and saved for each channel.

Later, when a different channel is processed for Ithresh, the saved data can be accessed and, based on that data, the algorithm may omit a stimulation signal and infer whether or not the channel would recruit at the given stimulation current.

There are two reasons the algorithm may omit a stimulation signal and report previous recruitment results. A stimulation signal may be omitted if the selected stimulation current would be a repeat of a previous stimulation. By way of example only, if a stimulation current of 1mA was applied to determine Iₜₕᵣₑₛₕ for one channel, and a stimulation at 1mA is later required to determine Iₜₕᵣₑₛₕ for another channel, the algorithm may omit the stimulation and report the previous results. If the specific stimulation current required has not previously been used, a stimulation signal may still be omitted if the results are already clear from the previous data. By way of example only, if a stimulation current of 2mA was applied to determine Iₜₕᵣₑₛₕ for a previous channel and the present channel did not recruit, when a stimulation at 1 mA is later required to determine Iₜₕᵣₑₛₕ for the present channel, the algorithm may infer from the previous stimulation that the present channel will not recruit at 1mA because it did not recruit at 2mA. The algorithm may therefore omit the stimulation and report the previous result.

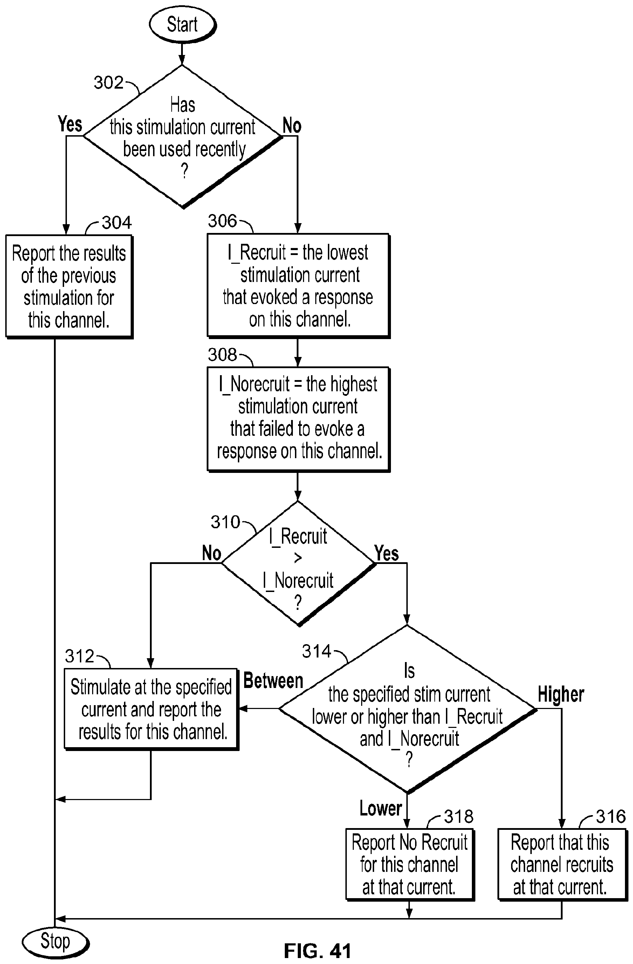

Fig. 41 illustrates (in flowchart form) a method by which the multi-channel threshold hunting algorithm determines whether to stimulate, or not stimulate and simply report previous results. The algorithm first determines if the selected stimulation current has already been used (step 302). If the stimulation current has been used, the stimulation is omitted and the results of the previous stimulation are reported for the present channel (step 304). If the stimulation current has not been used, the algorithm determines I_{recruit} (step 306) and I_{norecruit} (step 308) for the present channel. I_{recruit} is the lowest stimulation current that has recruited on the present channel. I_{norecruit} is the highest stimulation current that has failed to recruit on the present channel. The algorithm next determines whether I_{recruit} is greater than I_{norecruit} (step 310). An I_{recruit} that is not greater than I_{norecruit} is an indication that changes have occurred to Iₜₕᵣₑₛₕ on that channel. Thus, previous results may not be reflective of the present threshold state and the algorithm will not use them to infer the response to a given stimulation current. The algorithm will stimulate at the selected current and report the results for the present channel (step 312). If I_{recruit} is greater than I_{norecruit}, the algorithm determines whether the selected stimulation current is higher than I_{recruit}, lower than I_{norecruit}, or between I_{recruit} and I_{norecruit} (step 314). If the selected stimulation current is higher than I_{recruit}, the algorithm omits the stimulation and reports that the present channel recruits at the specified current (step 316). If the selected stimulation current is lower than I_{norecruit}, the algorithm infers that the present channel will not recruit at the selected current and reports that result (step 318). If the selected stimulation current falls between Recruit and Inorecruit, the result of the stimulation cannot be inferred and the algorithm stimulates at the selected current and reports the results for the present channel (step 312). This method may be repeated until _{Ithresh} has been determined for every active channel.

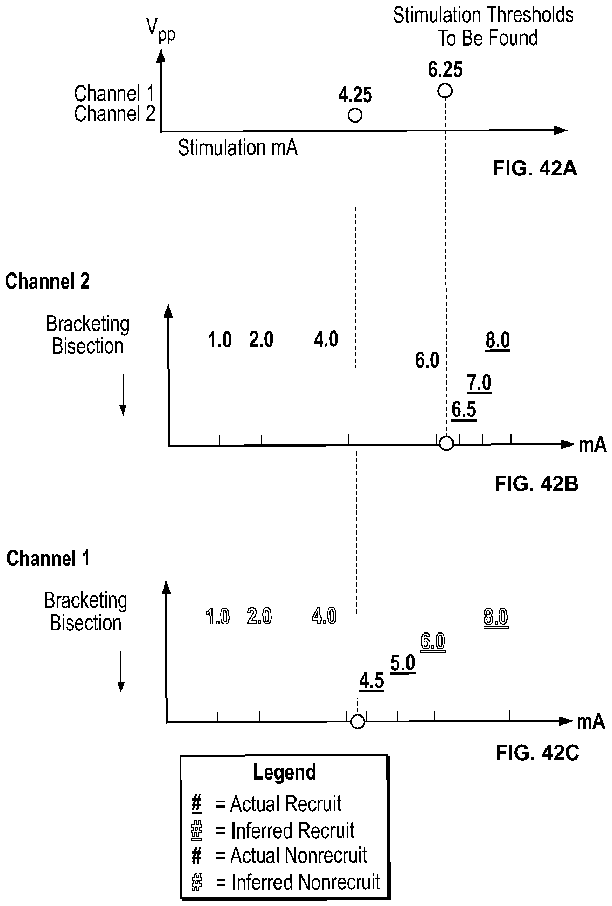

In the interest of clarity, Figs. 42 A-C demonstrate use of the multi-channel threshold hunting algorithm to determine _{Ithresh} on only two channels. It should be appreciated, however, that the multi-channel algorithm is not limited to finding thᵣₑₛh for two channels, but rather it may be used to find _{Ithresh} for any number of channels, such as (for example) eight channels. With reference to Fig. 42 A, channel 1 has an Ithresh to be found of 6.25 mA and channel 2 has an _{Ithresh} to be found of 4.25 mA. _{Ithresh} for channel 1 is found first as illustrated in Fig. 38 B, using the bracketing and bisection methods discussed above. Bracketing begins at the minimum stimulation current (for the purposes of example only) of 1 mA, As this is the first channel processed and no previous recruitment results exist, no stimulations are omitted. The stimulation current is doubled with each successive stimulation until a significant EMG response is evoked at 8 mA. The initial bracket of 4-8 mA is bisected, using the bisection method described above, until the stimulation threshold, Iₜₕᵣₑₛₕ, is contained within a final bracket separated by the selected width or resolution (again .25 mA). In this example, the final bracket is 6 mA-6.25 mA. Iₜₕᵣₑₛₕ may be defined as any point within the final bracket or as the midpoint of the final bracket (6.125 mA in this case). In either event, Iₜₕᵣₑₛₕ is selected and reported as Iₜₕᵣₑₛₕ for channel 1.

Once _{Ithresh} is found for channel 1, the algorithm turns to channel 2, as illustrated in Fig. 42 C. The algorithm begins to process channel 2 by determining the initial bracket, which is again 4-8 mA. All the stimulation currents required in the bracketing state were used in determining _{Ithresh} for channel 1. The algorithm refers back to the saved data to determine how channel 1 responded to the previous stimulations. From the saved data, the algorithm may infer that channel 2 will not recruit at stimulation currents of 1, 2, and 4 mA, and will recruit at 8 mA. These stimulations are omitted and the inferred results are displayed. The first bisection stimulation current selected in the bisection process (6 mA in this case), was previously used and, as such, the algorithm may omit the stimulation and report that channel 2 recruits at that stimulation current. The next bisection stimulation current selected (5 mA in this case) has not been previously used and, as such, the algorithm must determine whether the result of a stimulation at 5 mA may still be inferred. In the example shown, I_{recruit} and I_{norecruit} are determined to be 6 mA and 4 mA, respectively. Because 5 mA falls in between I_{recruit} and I_{norecruit}, the algorithm may not infer the result from the previous data and, as such, the stimulation may not be omitted. The algorithm then stimulates at 5 mA and reports that the channel recruits. The bracket is reduced to the lower half (making 4.50 mA the next bisection stimulation current). A stimulation current of 4.5 mA has not previously been used and, as such, the algorithm again determines I_{recruit} and I_{norecruit} (5 mA and 4 mA in this case). The selected stimulation current (4.5 mA) falls in between I_{recruit} an I_{norecruit} and, as such, the algorithm stimulates at 4.5 mA and reports the results. The bracket now stands at its final width of .25 mA (for the purposes of example only). Iₜₕᵣₑₛₕ may be defined as any point within the final bracket or as the midpoint of the final bracket (4.125 mA in this case). In either event, Iₜₕᵣₑₛₕ is selected and reported as Iₜₕᵣₑₛₕ for channel 2.

Although the multi-channel threshold hunting algorithm is described above as processing channels in numerical order, it will be understood that the actual order in which channels are processed is immaterial. The channel processing order may be biased to yield the highest or lowest threshold first (discussed below) or an arbitrary processing order may be used. Furthermore, it will be understood that it is not necessary to complete the algorithm for one channel before beginning to process the next channel, provided that the intermediate state of the algorithm is retained for each channel. Channels are still processed one at a time. However, the algorithm may cycle between one or more channels, processing as few as one stimulation current for that channel before moving on to the next channel. By way of example only, the algorithm may stimulate at 10mA while processing a first channel for Iₜₕᵣₑₛₕ. Before stimulating at 20 mA (the next stimulation current in the bracketing phase), the algorithm may cycle to any other channel and process it for the 10 mA stimulation current (omitting the stimulation if applicable). Any or all of the channels may be processed this way before returning to the first channel to apply the next stimulation. Likewise, the algorithm need not return to the first channel to stimulate at 20 mA, but instead may select a different channel to process first at the 20 mA level. In this manner, the algorithm may advance all channels essentially together and bias the order to find the lower threshold channels first or the higher threshold channels first. By way of example only, the algorithm may stimulate at one current level and process each channel in turn at that level before advancing to the next stimulation current level. The algorithm may continue in this pattern until the channel with the lowest Iₜₕᵣₑₛₕ is bracketed. The algorithm may then process that channel exclusively until Iₜₕᵣₑₛₕ is determined, and then return to processing the other channels one stimulation current level at a time until the channel with the next lowest Iₜₕᵣₑₛₕ is bracketed. This process may be repeated until Iₜₕᵣₑₛₕ is determined for each channel in order of lowest to highest Iₜₕᵣₑₛₕ. If Iₜₕᵣₑₛₕ for more than one channel falls within the same bracket, the bracket may be bisected, processing each channel within that bracket in turn until it becomes clear which one has the lowest Iₜₕᵣₑₛₕ. If it becomes more advantageous to determine the highest Iₜₕᵣₑₛₕ first, the algorithm may continue in the bracketing state until the bracket is found for every channel and then bisect each channel in descending order.

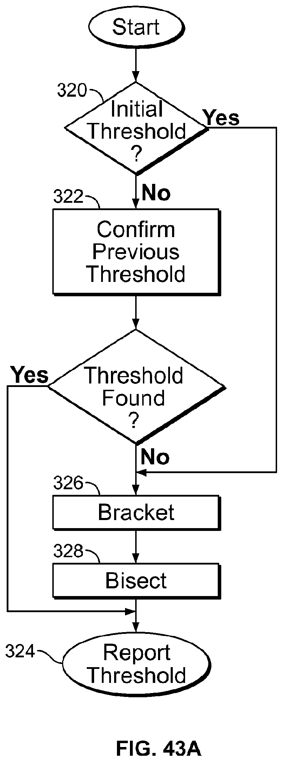

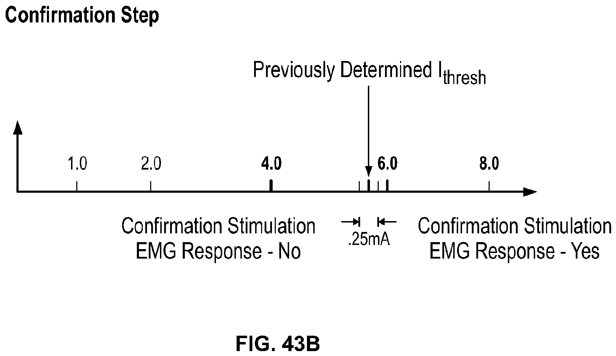

Figs. 43 A-B illustrates a further feature of the threshold hunting algorithm of the present invention, which advantageously provides the ability to further reduce the number of stimulations required to find Iₜₕᵣₑₛₕ when an Iₜₕᵣₑₛₕ value has previously been determined for a specific channel. In the event that a previous Iₜₕᵣₑₛₕ determination exists for a specific channel, the algorithm may begin by merely confirming the previous Iₜₕᵣₑₛₕ rather than beginning anew with the bracketing and bisection methods. The algorithm first determines whether it is conducting the initial threshold determination for the channel or whether there is a previous Iₜₕᵣₑₛₕ determination (step 320). If it is not the initial determination, the algorithm confirms the previous determination (step 322) as described below. If the previous threshold is confirmed, the algorithm reports that value as the present Iₜₕᵣₑₛₕ (step 324). If it is the initial Iₜₕᵣₑₛₕ determination, or if the previous threshold cannot be confirmed, then the algorithm performs the bracketing function (step 326) and bisection function (step 328) to determine Iₜₕᵣₑₛₕ and then reports the value (step 324).

Although the hunting algorithm is discussed herein in terms of finding Iₜₕᵣₑₛₕ (the lowest stimulation current that evokes a predetermined EMG response), it is contemplated that alternative stimulation thresholds may be useful in assessing the health of the spinal cord or nerve monitoring functions and may be determined by the hunting algorithm. By way of example only, the hunting algorithm may be employed by the system 10 to determine a stimulation voltage threshold. This is the lowest stimulation voltage necessary to evoke a significant EMG response. Bracketing, bisection and monitoring states are conducted as described above for each active channel, with brackets based on voltage being substituted for the current based brackets previously described. Moreover, although described above within the context of MEP monitoring, it will be appreciated that the algorithms described herein may also be used for determining the stimulation threshold (current or voltage) for any other EMG related functions, including but not limited to pedicle integrity (screw test), nerve detection, and nerve root retraction.

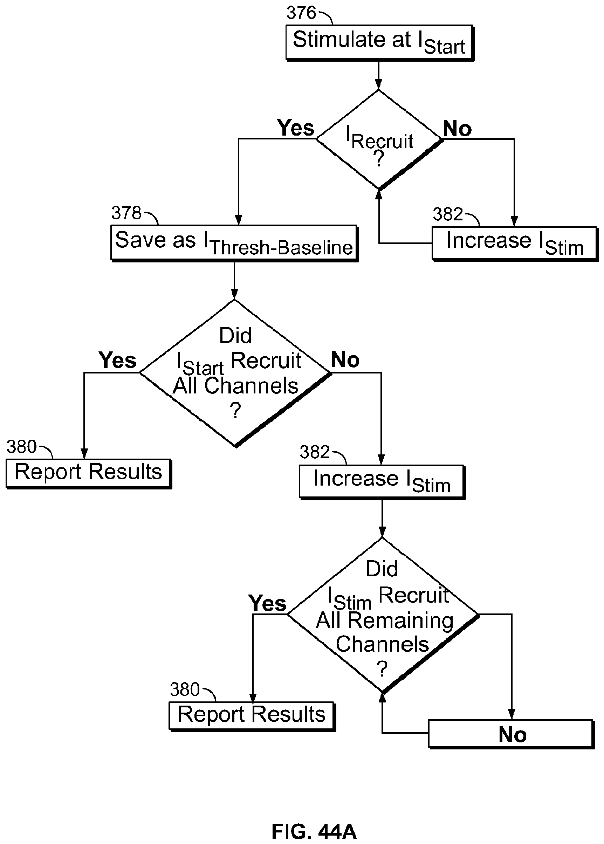

Figs. 44 A-B depict, by way of example only, the principles of a focused linear thresholding algorithm of the present invention used to quickly elicit a significant evoked neuromuscular response. The method for finding the evoked neuromuscular response utilizes a baseline ramping method and a subsequent ramping method to find Iₜₕᵣₑₛₕ. The baseline ramping method quickly finds stimulation intensity that generates a significant evoked neuromuscular response in each channel within a specified accuracy. The subsequent ramping method uses the baseline ramping results to find the minimum stimulation current threshold intensity that generates a significant neuromuscular response in each channel within a specified accuracy.

The baseline linear ramping method quickly finds a minimum stimulation intensity that generates responses by increasing Iₛₜᵢₘ in a linear fashion until Iₜₕᵣₑₛₕ is known within a specified accuracy for each channel. Stimulation begins at a starting stimulation current, such as (by way of example only) 100mA (step 376). It will be appreciated that the relevant current values depend in part on the function performed (e.g. high currents are used for TCNR and MEP and low currents are generally used for other functions) and the current values described here are for purposes of example only and may in actuality be adjusted to any scale.

The algorithm will increment each stimulation [level] from the preceding stimulation level by a fixed amount (e.g. 50mA) until a stimulation level recruits a baseline neuromuscular response (I_{thresh-baseline}) for each channel (step 382). The lowest stimulation level to recruit a baseline neuromuscular response in at least one channel is saved for recall in the subsequent focused linear ramping algorithm (step 378). Once baseline Iₜₕᵣₑₛₕ values for all channels have been defined, the results are reported (step 380) to the control unit 12.

According to some neurophysiologic monitoring modalities (e.g. MEP), it may be desirable to obtain baseline response in dual polarity stimulation configurations. In some implementations, the baseline linear ramping method includes the optional step of switching the polarity of stimulation at each stimulation intensity level and reporting the results of Iₜₕᵣₑₛₕ and the associated polarity at step 380.

While Iₜₕᵣₑₛₕ can be found for each channel as described above with respect to the linear ramping method every time, it requires a potentially large number of stimulations, each of which is associated with a specific time delay, which can add significantly to the response time. Done repeatedly, it could also add significantly to the overall time required to compete the surgical procedure, which may present added risk to the patient and added costs. To overcome this drawback, the focused linear thresholding algorithm includes a subsequent focused ramping method so as to quickly determine Iₜₕᵣₑₛₕ for each channel while minimizing the total number of stimulations, lowering the total energy delivered, and reducing the time required to perform such determinations.

The subsequent focused linear ramping method may reduce the number of stimulations required to find Iₜₕᵣₑₛₕ during subsequent stimulations after baseline, particularly when Iₜₕᵣₑₛₕ is being found for multiple channels. The algorithm does so by omitting stimulations for which the result is predictable from the baseline data already acquired. This permits the algorithm to proceed to the next step immediately, without the time delay associated with an unnecessary stimulation signal. The algorithm may then quickly find I_{thresh-subsequent} by using the lowest I_{thresh-baseline} (i.e. threshold stimulus intensity of the first channel to recruit at baseline) and increasing the stimulus intensity in a linear fashion until I_{thresh-subsequent} is known within a specified accuracy for each channel.

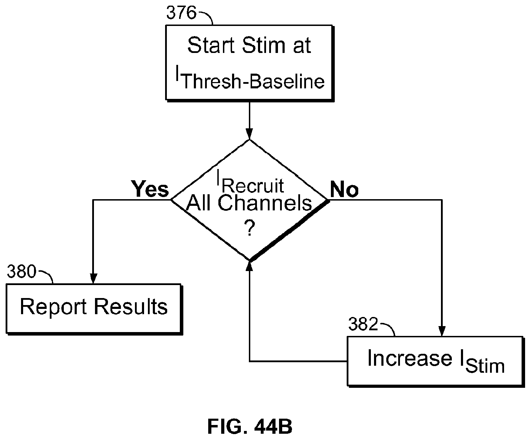

Referring back to the flowchart of Fig. 44B, in a subsequent TCNR test, the algorithm confirms the lowest baseline Iₜₕᵣₑₛₕ to recruit a response in at least one channel by stimulating at that Iₛₜᵢₘ (step 376). In those implementations including a switching of polarity, the algorithm confirms the baseline at polarity in which the lowest Iₜₕᵣₑₛₕ was obtained. If the Iₛₜᵢₘ at step 376 results in a response in all channels, the sequence stops and the results are reported to the control unit 12 (step 380). If the Iₛₜᵢₘ at step 380 does not result in a response in all channels, the stimulation is increased a predetermined amount (step 382) and the sequence is repeated until a response is repeated in each channel. Once subsequent Iₜₕᵣₑₛₕ values for all channels have been defined, the results are reported (step 380) to the control unit 12.

Although the focused linear thresholding algorithm is discussed herein in terms of finding Iₜₕᵣₑₛₕ (the lowest threshold stimulation intensity that evokes a predetermined EMG response), it is contemplated that alternative thresholds may be useful in assessing the health of the lumbar motor neural pathways, spinal cord, or nerve monitoring functions and may be determined by the algorithm. By way of example only, the algorithm may be employed by the system 10 do determined a stimulation voltage threshold Vstimₜₕᵣₑₛₕ. This is the lowest stimulation voltage (as opposed to the lowest stimulation current) necessary to evoke a significant EMG response, Vₜₕᵣₑₛₕ.

It will be appreciated that the focused linear thresholding algorithms described herein may also be used for determining the stimulation threshold (current or voltage) for any EMG-related functions, including but not limited to TCNR testing and MEP monitoring. Furthermore, it is to be understood that each neurophysiologic testing mode may include its own optimized stimulation profile, such that the term "stimulation" may comprise multiple meanings. By way of example only, TCNR stimulation may be a single pulse, such that the term "stimulation" may refer to a single pulse of stimulation energy whereas MEP stimulation may be a train of three to eight stimulation pulses such that the term "stimulation" may refer to a multiple train stimulation.

According to another broad aspect of the present disclosure, there is provided a method for monitoring the status of the motor neural pathway that includes the steps of: (a) stimulating the motor pathways in a transcutaneous and trans-abdominal fashion from a location superior (caudal, above) to the surgical site; (b) recording neurophysiologic responses evoked by that transcutaneous, trans-abdominal stimulation from one or more locations inferior (caudal, below) to the surgical site; and (c) comparing evoked responses over time to assess the health and status of the lower motor neural pathway. This particular method is useful in lumbar (as well as thoracic and thoracolumbar) spinal surgeries, where motor function may be at risk, to monitor and verify lumbar motor neural function throughout the procedure.

While this invention has been described in terms of a best mode for achieving this invention's objectives, it will be appreciated by those skilled in the art that variations may be accomplished in view of these teachings without deviating from the scope of the present invention. For example, the present invention may be implemented using any combination of computer programming software, firmware or hardware. As a preparatory step to practicing the invention or constructing an apparatus according to the invention, the computer programming code (whether software or firmware) according to the invention will typically be stored in one or more machine readable storage mediums such as fixed (hard) drives, diskettes, optical disks, magnetic tape, semiconductor memories such as ROMs, PROMs, etc., thereby making an article of manufacture in accordance with the invention. The article of manufacture containing the computer programming code is used by either executing the code directly from the storage device, by copying the code from the storage device into another storage device such as a hard disk, RAM, etc. or by transmitting the code on a network for remote execution. As can be envisioned by one of skill in the art, many different combinations of the above may be used and accordingly the present invention is not limited by the specified scope.

Any combination of the nerve monitoring methods described herein may be employed at any one time without departing from the scope of the present disclosure. For example, the transcutaneous, trans-abdominal nerve root stimulation method described herein may be used in conjunction with the monitoring method described above during surgical access with a surgical access system.

## Claims

1. A neurophysiologic monitoring system (10) comprising:
a control unit (12), a main display (34), a processing unit (36), a patient module (14), stimulation electrodes (22), and recording electrodes (24), and the system (10) further comprising a non-transitory computer-readable medium for monitoring the health of the lower motor neural pathway during a spinal procedure, comprising instructions stored thereon, that when executed by the processing unit (36), perform the steps of:
delivering, by the stimulation electrodes, a first transcutaneous, trans-abdominal stimulation signal to lumbar motor neural pathways superior to a surgical target site before establishing an operative corridor;
recording, by the recording electrodes, a first neuromuscular response to said first transcutaneous, trans-abdominal stimulation signal from one or more locations inferior to the surgical site;
delivering, by the stimulation electrodes, one or more subsequent transcutaneous, trans-abdominal stimulation signals to said lumbar motor neural pathways superior to said surgical target site during or after the establishing of the operative corridor;
recording, by the recording electrodes, a second neuromuscular response to said one or more subsequent transcutaneous, trans-abdominal stimulation signals from one or more locations inferior to the surgical site;
providing, by the recording electrodes, said first neuromuscular response and said second neuromuscular response to the control unit;
determining, by the control unit, one or more changes to a stimulation threshold intensity of said first transcutaneous, trans-abdominal stimulation signal and a stimulation threshold intensity of said one or more subsequent transcutaneous, trans-abdominal stimulation signals; and
based on the determination, converting, by the control unit, said first and second neuromuscular responses to a health status of neural structures for display;
**characterised in that** the stimulation electrodes (22) comprise a stimulating cathode electrode configured for placement posteriorly on a patient's body and an anode electrode configured for placement anteriorly on a patient's body, and wherein the anode electrode has a larger surface area than the cathode electrode.

2. The system of claim 1, wherein said first transcutaneous, trans-abdominal simulation signal is a fixed polarity single pulse signal.

3. The system of claim 1, wherein at least one of said one or more subsequent transcutaneous, trans-abdominal simulation signals is a fixed polarity single pulse signal.

4. The system of claim 1, wherein delivering said first transcutaneous, trans-abdominal stimulation signal comprises (i) emitting a stimulation current and (ii) increasing said stimulation current until said at least one neuromuscular response is elicited.

5. The system of claim 1, wherein delivering said one or more transcutaneous, trans-abdominal stimulation signals comprises (i) emitting a stimulation current and (ii) increasing said stimulation current until said at least one neuromuscular response is elicited.

6. The system of claim 1, wherein comparing said second stimulation threshold intensity to said first stimulation threshold intensity comprises: determining whether a change between (i) said first stimulation threshold intensity and (ii) said second stimulation threshold intensity exceeds a predetermined criteria.

7. The system of claim 1, further comprising providing an alert when a predetermined period of time has elapsed since delivering said one or more subsequent transcutaneous, trans-abdominal stimulation signals.

## Patentansprüche

1. Neurophysiologisches Überwachungssystem (10), umfassend:
eine Steuereinheit (12), eine Hauptanzeige (34), eine Verarbeitungseinheit (36), ein Patientenmodul (14), Stimulationselektroden (22) und Aufzeichnungselektroden (24), und wobei das System (10) ferner ein nicht-transitorisches computerlesbares Medium zur Überwachung des Zustands der unteren motorischen Nervenbahn während eines Wirbelsäuleneingriffs aufweist, das darauf gespeicherte Anweisungen aufweist, die, wenn sie von der Verarbeitungseinheit (36) ausgeführt werden, die folgenden Schritte durchführen:
- Abgeben eines ersten transkutanen, transabdominalen Stimulationssignals durch die Stimulationselektroden an lumbale motorische Nervenbahnen oberhalb einer chirurgischen Zielstelle, bevor ein operativer Korridor eingerichtet wird;
- Aufzeichnen einer ersten neuromuskulären Reaktion auf das erste transkutane, transabdominale Stimulationssignal durch die Aufzeichnungselektroden von einer oder mehreren Stellen unterhalb der Operationsstelle;
- Abgeben eines oder mehrerer nachfolgender transkutaner, transabdominaler Stimulationssignale durch die Stimulationselektroden an die lumbalen motorischen Nervenbahnen oberhalb der chirurgischen Zielstelle während oder nach der Einrichtung des operativen Korridors;
- Aufzeichnen durch die Aufzeichnungselektroden einer zweiten neuromuskulären Reaktion auf das eine oder die mehreren nachfolgenden transkutanen, transabdominalen Stimulationssignale von einer oder mehreren Stellen unterhalb der Operationsstelle;
- Vorsehen der ersten neuromuskulären Reaktion und der zweiten neuromuskulären Reaktion durch die Aufzeichnungselektroden an die Steuereinheit;
- Bestimmen, durch die Steuereinheit, einer oder mehrerer Änderungen einer Stimulationsschwellenintensität des ersten transkutanen, transabdomina- len Stimulationssignals und einer Stimulationsschwellenintensität des einen oder der mehreren nachfolgenden transkutanen, transabdominalen Stimulationssignale; und
- Umwandeln, auf der Basis der Bestimmung, der ersten und zweiten neuromuskulären Reaktionen durch die Steuereinheit in einen Gesundheitszustand der neuralen Strukturen zum Anzeigen;
- **dadurch gekennzeichnet, dass** die Stimulationselektroden (22) eine Stimulationskathodenelektrode aufweisen, die für eine Anordnung auf der Rückseite eines Patientenkörpers eingerichtet ist, und eine Anodenelektrode aufweisen, die für eine Anordnung auf der Vorderseite eines Patientenkörpers eingerichtet ist, und wobei die Anodenelektrode eine größere Oberfläche als die Kathodenelektrode aufweist.

2. System nach Anspruch 1, wobei das erste transkutane, transabdominale Simulationssignal ein Einzelimpulssignal mit fester Polarität ist.

3. System nach Anspruch 1, wobei zumindest eins des einen oder der mehreren nachfolgenden transkutanen, transabdominalen Simulationssignale ein Einzelimpulssignal mit fester Polarität ist.

4. System nach Anspruch 1, wobei ein Abgeben des ersten transkutanen, transabdominalen Stimulationssignal (i) ein Aussenden eines Stimulationsstroms und (ii) ein Erhöhen des Stimulationsstroms aufweist, bis die zumindest eine neuromuskuläre Reaktion hervorgerufen wird.

5. System nach Anspruch 1, wobei ein Abgeben des einen oder der mehreren transkutanen, transabdominalen Stimulationssignale (i) ein Aussenden eines Stimulationsstroms und (ii) ein Erhöhen des Stimulationsstroms aufweist, bis die zumindest eine neuromuskuläre Reaktion hervorgerufen wird.

6. System nach Anspruch 1, wobei ein Vergleichen der zweiten Stimulationsschwellenintensität mit der ersten Stimulationsschwellenintensität aufweist:
- Bestimmen, ob eine Änderung zwischen (i) der ersten Stimulationsschwellenintensität und (ii) der zweiten Stimulationsschwellenintensität ein vorbestimmtes Kriterium überschreitet.

7. System nach Anspruch 1, das ferner ein Vorsehen eines Alarms aufweist, wenn eine vorbestimmte Zeitdauer seit der Abgabe des einen oder der mehreren nachfolgenden transkutanen transabdominalen Stimulationssignale verstrichen ist.

## Revendications

1. Système de surveillance neurophysiologique (10) comprenant:
une unité de commande (12), un affichage principal (34), une unité de traitement (36), un module patient (14), des électrodes de stimulation (22) et des électrodes d'enregistrement (24), et le système (10) comprenant en outre un support lisible par ordinateur non transitoire pour surveiller la santé de la voie neuronale motrice inférieure pendant une opération vertébrale, comprenant des instructions stockées sur celui-ci, qui, lorsqu'elles sont exécutées par l'unité de traitement (36), réalisent les étapes de:
délivrer, par les électrodes de stimulation, un premier signal de stimulation trans-abdominale transcutanée aux voies neuronales motrices lombaires supérieures à un site cible chirurgical avant l'établissement d'un couloir opératoire;
enregistrer, par les électrodes d'enregistrement, une première réponse neuromusculaire audit premier signal de stimulation trans-abdominale transcutanée provenant d'un ou plusieurs emplacements inférieurs au site chirurgical;
délivrer, par les électrodes de stimulation, un ou plusieurs signaux de stimulation trans-abdominale transcutanée subséquents auxdites voies neuronales motrices lombaires supérieures audit site cible chirurgical pendant ou après l'établissement du couloir opératoire;
enregistrer, par les électrodes d'enregistrement, une seconde réponse neuromusculaire auxdits un ou plusieurs signaux de stimulation trans-abdominale transcutanée subséquents provenant d'un ou plusieurs emplacements inférieurs au site chirurgical;
fournir, par les électrodes d'enregistrement, ladite première réponse neuromusculaire et ladite seconde réponse neuromusculaire à l'unité de commande;
déterminer, par l'unité de commande, un ou plusieurs changements d'une intensité seuil de stimulation dudit premier signal de stimulation trans-abdominale transcutanée et d'une intensité seuil de stimulation desdits un ou plusieurs signaux de stimulation trans-abdominale transcutanée subséquents; et
sur la base de la détermination, convertir, par l'unité de commande, lesdites première et seconde réponses neuromusculaires en un état de santé de structures neuronales pour l'affichage;
**caractérisé en ce que** les électrodes de stimulation (22) comprennent une électrode cathodique de stimulation configurée pour la mise en place postérieurement sur le corps d'un patient et une électrode anodique configurée pour la mise en place antérieurement sur le corps d'un patient, et dans lequel l'électrode anodique a une surface plus grande que l'électrode cathodique.

2. Système selon la revendication 1, dans lequel ledit premier signal de simulation trans-abdominale transcutanée est un signal à une seule impulsion de polarité fixée.

3. Système selon la revendication 1, dans lequel au moins l'un desdits un ou plusieurs signaux de simulation trans-abdominale transcutanée subséquents est un signal à une seule impulsion de polarité fixée.

4. Système selon la revendication 1, dans lequel la délivrance dudit premier signal de stimulation trans-abdominale transcutanée comprend (i) l'émission d'un courant de stimulation et (ii) l'augmentation dudit courant de stimulation jusqu'à ce que ladite au moins une réponse neuromusculaire soit déclenchée.

5. Système selon la revendication 1, dans lequel la délivrance desdits un ou plusieurs signaux de stimulation trans-abdominale transcutanée comprend (i) l'émission d'un courant de stimulation et (ii) l'augmentation dudit courant de stimulation jusqu'à ce que ladite au moins une réponse neuromusculaire soit déclenchée.

6. Système selon la revendication 1, dans lequel la comparaison de ladite seconde intensité seuil de stimulation à ladite première intensité seuil de stimulation comprend:
la détermination de ce qu'un changement entre (i) ladite première intensité seuil de stimulation et (ii) ladite seconde intensité seuil de stimulation dépasse un critère prédéterminé.

7. Système selon la revendication 1, comprenant en outre la fourniture d'une alerte lorsqu'une période de temps prédéfinie s'est écoulée depuis la délivrance desdits un ou plusieurs signaux de stimulation trans-abdominale transcutanée subséquents.
